# FASCICULE DE BREVET EUROPEEN

(11) **EP 4 025 630 B1**
(45) Date de publication et mention de la délivrance du brevet: **24.12.2025**
(21) Numéro de dépôt: 20764392.5
(22) Date de dépôt: 03.09.2020
(51) Int. Cl.: C08G 83/00, C08F 283/06, C07K 17/08, C08G 69/10

(54) **PROCÉDÉ DE PRÉPARATION DE POLYMÈRES ET COPOLYMÈRES CONTRÔLÉS À BASE DE PEPTIDES EN SOLUTION AQUEUSE**
VERFAHREN ZUR HERSTELLUNG VON KONTROLLIERTEN PEPTIDBASIERTEN POLYMEREN UND COPOLYMEREN IN WÄSSRIGER LÖSUNG
METHOD FOR PREPARING CONTROLLED PEPTIDE-BASED POLYMERS AND COPOLYMERS IN AN AQUEOUS SOLUTION

(30) Priorité: 03.09.2019 FR 1909678
(43) Date de publication de la demande: 13.07.2022
(73) Titulaire: Université de Bordeaux, 33000 Bordeaux (FR); Institut Polytechnique de Bordeaux, 33400 Talence (FR); Centre National de la Recherche Scientifique, 75016 Paris (FR)
(72) Inventeur: LECOMMANDOUX, Sébastien, 33610 CANEJAN (FR); BONDUELLE, Colin, 33380 Marcheprime (FR); GARANGER, Elisabeth, 33400 TALENCE (FR); GRAZON, Chloé, 33000 BORDEAUX (FR)
(74) Mandataire: Lavoix
(86) Numéro de dépôt international: PCT/EP2020/074533
(87) Numéro de publication internationale: WO 2021/043865

(56) Documents cités:
- VARLAS SPYRIDON ET AL: "Poly(sarcosine)-Based Nano-Objects with Multi-Protease Resistance by Aqueous Photoinitiated Polymerization-Induced Self-Assembly (Photo-PISA)", BIOMACROMOLECULES, vol. 19, no. 11, 23 October 2018 (2018-10-23), US, pages 4453 - 4462, XP055970202, ISSN: 1525-7797, Retrieved from the Internet <URL:https://pubs.acs.org/doi/pdf/10.1021/acs.biomac.8b01326> DOI: 10.1021/acs.biomac.8b01326
- IWATSUKI ET AL: "Sudies on Chitin. 13 New Polysaccharide/Polypeptide Hybrid Materials Based on Chitin and Poly(7-methyl L-glutamate)", MACROMOLECULES MACROMOLECULES J. AM. CHEM. SOC. J. AM. CHEM. SOC. J. AM. CHEM. SOC. PROG. POLYM. SCI. JPN. MACROMOLECULES MACROMOLECULES MACROMOLECULES MACROMOLECULES J. MACROMOLECULES, 1 January 1988 (1988-01-01), pages 1579 - 1583, XP055694800, Retrieved from the Internet <URL:https://pubs.acs.org/doi/abs/10.1021/ma00184a007>
- KEISUKE KURITA ET AL: "Studies on Chitin 11. Graft Copolymerization of y-Methyl L-Glutamate NCA onto Water-Soluble Chitin", POLYMER BULLETIN, 1 January 1985 (1985-01-01), pages 511 - 514, XP055694801, Retrieved from the Internet <URL:https://link.springer.com/article/10.1007/BF00271608>
- JINHUI JIANG ET AL: "Ring-Opening Polymerization of N -Carboxyanhydride-Induced Self-Assembly for Fabricating Biodegradable Polymer Vesicles", ACS MACRO LETTERS, vol. 8, no. 10, 15 October 2019 (2019-10-15), pages 1216 - 1221, XP055694933, ISSN: 2161-1653, DOI: 10.1021/acsmacrolett.9b00606
- JINHUI JIANG ET AL: "Supporting Information Ring-Opening Polymerization of N-Carboxyanhydride- Induced Self-Assembly for Fabricating Biodegradable Polymer Vesicles", 11 September 2019 (2019-09-11), XP055730134, Retrieved from the Internet <URL:https://pubs.acs.org/doi/suppl/10.1021/acsmacrolett.9b00606/suppl_file/mz9b00606_si_001.pdf> [retrieved on 20200911]
- NICHOLAS J. WARREN ET AL: "Polymerization-Induced Self-Assembly of Block Copolymer Nano-objects via RAFT Aqueous Dispersion Polymerization", JOURNAL OF THE AMERICAN CHEMICAL SOCIETY, vol. 136, no. 29, 23 July 2014 (2014-07-23), US, pages 10174 - 10185, XP055394554, ISSN: 0002-7863, DOI: 10.1021/ja502843f
- TAKUYA MIYAZAKI ET AL: "One-Pot Synthesis of PEG-Poly(amino acid) Block Copolymers Assembling Polymeric Micelles with PEG-Detachable Functionality", ACS BIOMATERIALS SCIENCE & ENGINEERING, vol. 5, no. 11, 22 January 2019 (2019-01-22), US, pages 5727 - 5733, XP055694799, ISSN: 2373-9878, DOI: 10.1021/acsbiomaterials.8b01549

## Description

La présente invention a pour objet un procédé de préparation de polymères et copolymères contrôlés à base de peptides en solution aqueuse, ainsi que les produits tels qu'obtenus.

L'auto-assemblage de copolymères amphiphiles est une stratégie prometteuse pour concevoir des nanomatériaux de pointe dotés de fonctions uniques. Parmi les copolymères amphiphiles, les polypeptides constituent une classe émergente de biomatériaux qui sont utilisés comme vecteurs d'actifs dans des applications pharmaceutiques ou cosmétiques. Jusqu'à présent, le procédé le plus économique et le plus efficace pour préparer des nanomatériaux à base de polypeptides amphiphiles est un procédé qui nécessite plusieurs étapes, incluant en particulier la polymérisation par ouverture de cycle de monomères de N-carboxyanhydride (ROP ou ROPISA).

Cette polymérisation contrôlée fait appel aux réactifs les plus simples mais souffre toujours de limitations importantes, notamment 1) de fastidieuses étapes de purification du monomère N-carboxyanhydride ; 2) une sensibilité significative à l'eau et à l'humidité et 3) une mise en œuvre dans des solvants organiques toxiques tels que le DMF qui doivent ensuite être éliminés. La conception de nanoparticules à partir de polypeptides amphiphiles implique au moins une seconde étape de formulation, la nanoprécipitation, qui consiste à ajouter un non-solvant pour le segment hydrophobe à une solution de copolymère dans un solvant commun aux deux blocs. L'étape de nanoprécipitation est généralement réalisée à partir de solvants organiques toxiques ou volatiles, dans des conditions relativement diluées (<1% en poids) et est sensible aux problèmes liés au changement d'échelle, ce qui nuit généralement à une approbation réglementaire du procédé.

L'auto-assemblage induit par polymérisation (PISA) est une approche simple et robuste pour accéder à des polymères amphiphiles avec l'avantage d'obtenir simultanément des nanoparticules de ces mêmes polymères. La méthode PISA implique la croissance *in situ* d'une chaîne polymère amphiphile vivante qui s'auto-assemble de façon spontanée dans des nanostructures. Jusqu'à présent, la méthode PISA a été mise en œuvre en utilisant des procédés de polymérisation radicalaire (RAFT, ATRP, NMP, CMP, TERP), soit en dispersion, soit en émulsion.

La présente invention a pour but de fournir des nano-objets fonctionnalisés, en une seule étape de préparation.

Un autre but de la présente invention consiste à fournir un procédé « one pot » simple et rapide en une seule étape en milieu aqueux pour obtenir des copolymères peptidiques amphiphiles, et ce sans nécessiter de purification ultérieure.

La présente invention a également pour but de fournir un procédé de polymérisation avec des cinétiques de polymérisation très rapides, permettant d'obtenir des polymères fonctionnels, bioassimilables, biocompatibles et biodégradables.

Ainsi, la présente invention concerne un procédé de préparation « one pot » d'une solution aqueuse de nanoparticules de copolymères à blocs amphiphiles et comprenant des motifs polypeptidiques, ledit procédé comprenant au moins une étape (E1), dans un solvant aqueux dénué de solvant organique, consistant à mettre en présence :
- au moins un polymère hydrophile (P1) comprenant au moins une fonction amine, et
- au moins un monomère N-carboxyanhydride d'α-acide aminé (NCA) hydrophobe dans lequel la température de l'étape (E1) est comprise de -10°C à 80°C, ledit procédé étant un procédé d'auto-assemblage induit par polymérisation par ouverture de cycle de N-carboxyanhydride.

Le procédé de l'invention permet donc d'obtenir une solution aqueuse de nanoparticules de copolymères à blocs amphiphiles à base de polypeptides, et ce en milieu aqueux.

Il est basé sur un procédé d'auto-assemblage induit par polymérisation (dit PISA). Ainsi, en adaptant le procédé PISA aux monomères NCA, les inventeurs ont constaté de façon fortuite que l'auto-assemblage spontané d'un procédé PISA permettait de les protéger de l'hydrolyse.

La présente invention concerne donc la préparation de polypeptides amphiphiles dans des solutions aqueuses par polymérisation par ouverture de cycle.

Le procédé de l'invention est un procédé ne nécessitant pas l'utilisation d'un solvant organique. Selon l'invention, le procédé est effectué en l'absence de tout solvant organique.

Selon l'invention, le solvant aqueux ne comprend pas de solvant organique.

La présente invention concerne donc un procédé de préparation de polymères et copolymères contrôlés à base de peptides en solution aqueuse ainsi que leur auto-formulation spontanée durant ce même procédé permettant ainsi de former des nanoparticules stables et fonctionnelles en une seule étape de préparation, qui pourront entrer par exemple et de façon non-exclusive dans la préparation de compositions pharmaceutiques ou cosmétiques.

Le procédé de l'invention consiste à préparer des copolymères amphiphiles polypeptidiques sans solvant organique, rapide et contrôlé, permettant par ailleurs la formation concomitante de nanoparticules, le tout à des taux d'extraits secs supérieurs à 10% massique, permettant ainsi de réduire la complexité des procédés déjà connus. En particulier, la présente invention concerne un procédé de préparation de copolymères polypeptidiques amphiphiles et d'auto-formulation spontanée plus rapide en mettant en œuvre ces deux étapes de façon concomitante et sans purification, en milieu aqueux.

Comme mentionné ci-dessus, le procédé de l'invention comprend la mise en œuvre d'au moins un monomère NCA hydrophobe.

Tout monomère NCA hydrophobe peut être utilisé. Selon la nature du monomère NCA utilisé, il est possible de le modifier, notamment via un groupe protecteur hydrophobe, pour le rendre hydrophobe.

Selon un mode de réalisation, le monomère N-carboxyanhydride d'α-acide aminé hydrophobe répond à la formule (I) suivante : 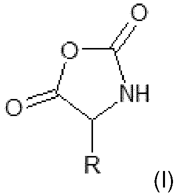 dans laquelle R représente la chaîne latérale d'un α-acide aminé, naturel ou modifié, hydrophobe, éventuellement protégé.

Lorsque le monomère NCA utilisé est un NCA d'α-acide aminé hydrophile (comprenant des fonctions OH, COOH ou NH₂ notamment), alors le groupe R susmentionné comprend un groupement protecteur hydrophobe, afin de rendre hydrophobe ledit monomère.

Cette protection n'est pas nécessaire lorsque le NCA utilisés est une NCA d'un α-acide aminé hydrophobe par nature.

A titre de monomère N-carboxyanhydride d'α-acide aminé hydrophobe, on utilise notamment les composés ci-dessous :

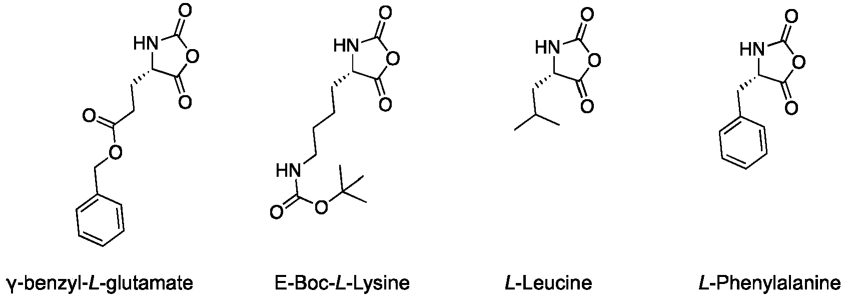

On peut de préférence citer les monomères NCA du γ-benzyl-*L*-glutamate, du ε-Boc-*L*-Lysine, de la L-leucine ou de la L-Phénylalanine.

Comme mentionné ci-dessus, le procédé de l'invention comprend la mise en œuvre d'au moins un polymère hydrophile (P1) comprenant au moins une fonction amine. Ce polymère hydrophile sert de macro-amorceur.

Selon un mode de réalisation, le polymère (P1) est choisi dans le groupe constitué des polyéthers, des polyesters, des poly(met)arylates, des polysaccharides, des polypeptides, des polypeptoides, des dérivés d'ADN et des protéines, en particulier les polymères à base d'élastine (elastin-like polypeptides (ELP)) comprenant au moins une fonction amine, et est de préférence choisi parmi les poly(oxyde d'éthylène)s comprenant au moins une fonction amine.

Préférentiellement, le polymère (P1) est un PEG.

De préférence, le polymère hydrophile (P1) a un poids moléculaire supérieur à 500 g/mol et a, de préférence, un poids moléculaire compris entre 2000 g/mol et 10000 g/mol.

De préférence, le polymère (P1) répond à la formule suivante : 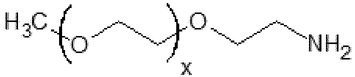 dans laquelle x est compris de 16 à 500.

Selon le procédé de l'invention, le produit de départ peut être assimilé à une suspension, à savoir un milieu hétérogène opaque, et le produit final obtenu est de préférence sous forme d'une solution transparente homogène.

Le procédé de l'invention permet donc avantageusement de transformer un milieu très hétérogène, notamment un solide dispersé dans de l'eau, en une solution de nanoparticules bien définies à base de copolymères amphiphiles d'architecture macromoléculaire contrôlée.

Le procédé de l'invention est un procédé « one pot », c'est-à-dire un procédé dans lequel les réactifs subissent une ou plusieurs réactions successives ou simultanées, mais dans un seul mélange réactionnel.

Selon un mode de réalisation, dans le procédé de l'invention, le solvant aqueux est de l'eau ou un tampon.

Par exemple, le solvant aqueux est de l'eau additionnée d'une solution tampon.

Selon un mode de réalisation, le solvant aqueux comprend en outre une solution tampon comprenant un sel à des concentrations allant de 0,01 M à 1 M, notamment choisi dans le groupe constitué des solutions d'hydrogénocarbonate de sodium et des solutions tampons phosphates.

De préférence, le procédé de l'invention met en œuvre une solution aqueuse de NaHCO₃.

De préférence, le pH du solvant aqueux est compris entre 2 à 12, et en particulier entre 7 et 10.

Selon l'invention, la température de l'étape (E1) est comprise de -10°C à 80°C, et préférentiellement de 0°C à 4°C.

Selon un mode de réalisation, l'étape (E1) est effectuée sous agitation à partir d'une dispersion du monomère N-carboxyanhydride d'α-acide aminé hydrophobe.

Le procédé de l'invention peut également être appliqué pour obtenir des copolymères multiblocs.

Ainsi, selon un mode de réalisation, la solution aqueuse de nanoparticules de copolymères à blocs amphiphiles obtenue à l'issue de l'étape (E1) est mise en contact ultérieurement avec un deuxième monomère N-carboxyanhydride d'α-acide aminé hydrophobe, identique ou différent de celui de l'étape (E1), ce qui permet d'obtenir une solution aqueuse de nanoparticules modifiées de copolymères à blocs amphiphiles modifiés.

Ainsi, selon ce mode de réalisation, il est possible d'obtenir des copolymères multiblocs, à gradient ou statistique lorsqu'on ajoute au moins un deuxième monomère N-carboxyanhydride d'α-acide aminé hydrophobe différent de celui de l'étape (E1).

Le procédé de l'invention peut être appliqué pour obtenir des nanoparticules de préférence cœur-couronne, de préférence allongées, de préférence rigides, de préférence d'une taille allant de 2 nm à 1 µm. Ainsi, selon ce mode de réalisation, il est possible d'obtenir des nanoparticules anisotropes ayant des propriétés optiques.

Des nanoparticules de copolymères à blocs amphiphiles et comprenant des motifs polypeptidiques, sont obtenues selon le procédé tel que défini ci-dessus.

Des nanoparticules de copolymères à blocs amphiphiles et comprenant des motifs polypeptidiques, peuvent également être obtenues selon le procédé tel que défini ci-dessus, lesdites nanoparticules ayant une structure cœur-écorce et une taille de particules comprise de 2 nm à 1 µm.

Une composition aqueuse comprenant des nanoparticules telles que définies ci-dessus peut également être obtenue, la teneur en poids desdites nanoparticules étant d'au moins 2% par rapport au poids de ladite composition aqueuse, et de préférence comprise entre 2% en poids et 15% en poids par rapport au poids de ladite composition aqueuse.

Ce taux de solides est mesuré après la polymérisation et après purification des sels par dialyse en réalisant une lyophilisation (comparaison de la masse des résidus avec la masse avant lyophilisation).

### DESCRIPTION DES FIGURES

La Figure 1 concerne la chromatographie d'exclusion stérique réalisée dans le DMF (+1 mg/mL de LiBr) du copolymère PEG₅ₖ-b-PBLG de l'exemple 1. Gauche : détection RI, Droite : détection par absorbance.
La Figure 2 représente le spectre RMN ¹H réalisé dans le CDCl₃ + 15% TFA du copolymère PEG₅ₖ-b-PBLG de l'exemple 1.
La Figure 3 représente la distribution en intensité diffusée du diamètre hydrodynamique (Dh) des nanoparticules de PEG₅ₖ-b-PBLG de l'exemple 1 (dans l'eau ultra-pure).
La Figure 4 représente des clichés de Microscopie Electronique à Transmission de nanoparticules cryogénisées (Cryo-TEM) : nanoparticules du copolymère PEG₅ₖ-b-PBLG de l'exemple 1.
La Figure 5 représente la chromatographie d'exclusion stérique réalisée dans le DMF (+1 mg/mL de LiBr) du copolymère PEG₅ₖ-b-PBLG de l'exemple 2. Gauche : détection RI, Droite : détection par absorbance.
La Figure 6 représente le spectre RMN ¹H réalisé dans le CDCl₃ + 15% TFA du copolymère PEG₅ₖ-b-PBLG de l'exemple 2.
La Figure 7 représente un cliché de Cryo-TEM des nanoparticules du copolymère PEG₅ₖ-b-PBLG de l'exemple 2.
La Figure 8 représente la chromatographie d'exclusion stérique réalisée dans le DMF (+1 mg/mL de LiBr) du copolymère PEG₅ₖ-b-PBLG de l'exemple 3. Gauche : détection RI, Droite : détection par absorbance.
La Figure 9 représente le spectre RMN ¹H réalisé dans le CDCl₃ + 15% TFA du copolymère PEG₅ₖ-b-PBLG de l'exemple 3.
La Figure 10 représente la distribution (en intensité diffusée) du diamètre hydrodynamique (Dh) des nanoparticules de PEG₅ₖ-b-PBLG de l'exemple 3 (dans l'eau ultra-pure).
La Figure 11 représente la chromatographie d'exclusion stérique réalisée dans le DMF (+1 mg/mL de LiBr) du copolymère PEG₅ₖ-b-PBLG de l'exemple 4. Gauche : détection RI, Droite : détection par absorbance.
La Figure 12 représente le spectre RMN ¹H réalisé dans le CDCl₃ + 15% TFA du copolymère PEG₅ₖ-b-PBLG de l'exemple 4.
La Figure 13 représente la distribution (en intensité diffusée) du diamètre hydrodynamique (Dh) des nanoparticules de PEG₅ₖ-b-PBLG de l'exemple 4 (dans l'eau ultra-pure).
La Figure 14 représente un cliché de Cryo-TEM des nanoparticules du copolymère PEG₅ₖ-b-PBLG de l'exemple 4.
La Figure 15 concerne la chromatographie d'exclusion stérique réalisée dans le DMF (+1 mg/mL de LiBr) du copolymère PEG₂ₖ-b-PBLG de l'exemple 5. Détection RI en noir et gris et détection par absorbance en trait gris pointillé.
La Figure 16 représente la distribution (en intensité diffusée) du diamètre hydrodynamique (Dh) des nanoparticules de PEG₂ₖ-b-PBLG de l'exemple 5 (dans l'eau ultra-pure).
La Figure 17 représente la chromatographie d'exclusion stérique réalisée dans le DMF (+1 mg/mL de LiBr) du copolymère PEG₁₀ₖ-b-PBLG de l'exemple 6. Gauche : détection RI, Droite : détection par absorbance
La Figure 18 représente le spectre RMN ¹H réalisé dans le CDCl₃ + 15% TFA du copolymère PEG₁₀ₖ-b-PBLG de l'exemple 6.
La Figure 19 représente un cliché de Cryo-TEM des nanoparticules du copolymère PEG₁₀ₖ-b-PBLG de l'exemple 6.
La Figure 20 représente A) la chromatographie d'exclusion stérique réalisée dans le DMF (+1 mg/mL de LiBr) du copolymère ELPM40-b-PBLG de l'exemple 7 (Détection RI) ; B) un cliché TEM (coloration acétate uranyle) des nanoparticules du copolymère ELPM40-b-PBLG de l'exemple 7.
La Figure 21 représente la chromatographie d'exclusion stérique réalisée dans le DMF (+1 mg/mL de LiBr) du copolymère PEG₅ₖ-b-PLys de l'exemple 8. Détection RI.
La Figure 22 représente le spectre RMN ¹H réalisé dans le DMF-d6 du copolymère PEG₅ₖ-b-PLys de l'exemple 8.
La Figure 23 représente la distribution (en intensité diffusée) du diamètre hydrodynamique (Dh) des nanoparticules de PEG₅ₖ-b-PLys de l'exemple 8 (dans l'eau ultra-pure).
La Figure 24 représente un cliché de Cryo-TEM des nanoparticules du copolymère PEG₅ₖ-b-PLys de l'exemple 8.
La Figure 25 représente la chromatographie d'exclusion stérique réalisée dans le DMF (+1 mg/mL de LiBr) du copolymère PEG₅ₖ-b-PLys-b-PBLG de l'exemple 9. Détection RI.
La Figure 26 représente A) la chromatographie d'exclusion stérique réalisée dans le DMF (+1 mg/mL de LiBr) du copolymère PLeu-b-PBLG de l'exemple 10 (Détection RI) ; B) un cliché TEM (coloration acétate uranyle) des nanoparticules du copolymère PLeu-b-PBLG de l'exemple 10.
La Figure 27 représente A) la chromatographie d'exclusion stérique réalisée dans le DMF (+1 mg/mL de LiBr) du copolymère PPhe-b-PBLG de l'exemple 11 (Détection RI) ; B) un cliché TEM (coloration acétate uranyle) des nanoparticules du copolymère PPhe-b-PBLG de l'exemple 11.
La Figure 28 représente A) la chromatographie d'exclusion stérique réalisée dans le DMF (+1 mg/mL de LiBr) du copolymère PEG₅ₖ-b-PBLG-b-PEG₅ₖ de l'exemple 12 (Détection RI) ; B) un cliché TEM (coloration acétate uranyle) des nanoparticules du copolymère PEG₅ₖ-b-PBLG-b-PEG₅ₖ de l'exemple 12.
La Figure 29 représente A) la chromatographie d'exclusion stérique réalisée dans le DMF (+1 mg/mL de LiBr) du copolymère PEG-4arm-b-(PBLG)₄ de l'exemple 13 (Détection RI) ; B) un cliché TEM (coloration acétate uranyle) des nanoparticules du copolymère PEG-4arm-b-(PBLG)₄ de l'exemple 13.
La Figure 30 représente A) la chromatographie d'exclusion stérique réalisée dans le DMF (+1 mg/mL de LiBr) du copolymère PSar-b-PBLG de l'exemple 14 (Détection RI) ; B) un cliché TEM (coloration acetate uranyle) des nanoparticules du copolymère PSar-b-PBLG de l'exemple 14.
La Figure 31 représente A) la chromatographie d'exclusion stérique réalisée dans H₂0 (Juanito buffer) du copolymère PGA-b-PBLG de l'exemple 15 après déprotection (Détection RI) ; B) un cliché TEM (coloration acetate uranyle) des nanoparticules du copolymère PGA-b-PBLG de l'exemple 15.
La Figure 32 représente A) une analyse infra-rouge de l'exemple 16 (poudre) ; B) un cliché TEM (coloration acetate uranyle) des nanoparticules du copolymère de l'exemple 16.
La Figure 33 représente la chromatographie d'exclusion stérique réalisée dans le DMF (+1 mg/mL de LiBr) du copolymère PEG₅ₖ-b-PBLG de l'exemple 17 (Détection RI);
La Figure 34 représente A) un cliché TEM (coloration acétate uranyle) des nanoparticules du copolymère de l'exemple 18 ; B) la distribution (en intensité diffusée) du diamètre hydrodynamique (Dh) des nanoparticules de l'exemple 18 (dans l'eau ultra-pure) ; C) un gel d'électrophorèse montrant la formation du copolymère de l'exemple 18.

### EXEMPLES

### Exemple 1 : Synthèse concomitante d'un copolymère diblocs peptidique amphiphile poly(éthylène glycol)₅ₖ-block-poly(γ-benzyl-L-glutamate) et de leur nanoparticules correspondantes

Le monomère γ-Benzyl-*L*-glutamate *N*-carboxyanhydride (BLG-NCA) est un réactif chimique commercial qui est distribué par l'entreprise ISOCHEM. Le PEG₅ₖ-NH₂ (Mp = 5516 Da, Ð = 1.02) est distribué par l'entreprise RAPP Polymer. Les autres réactifs sont distribués par l'entreprise Sigma-Aldrich.

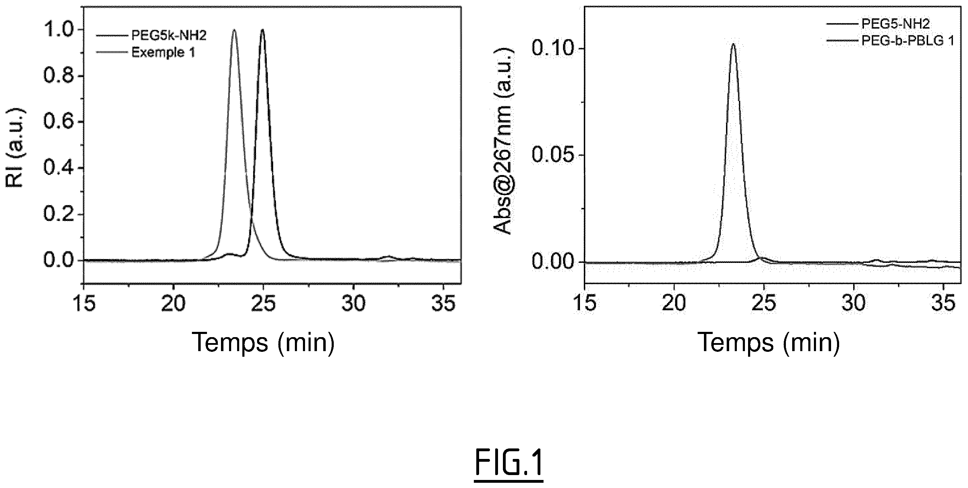

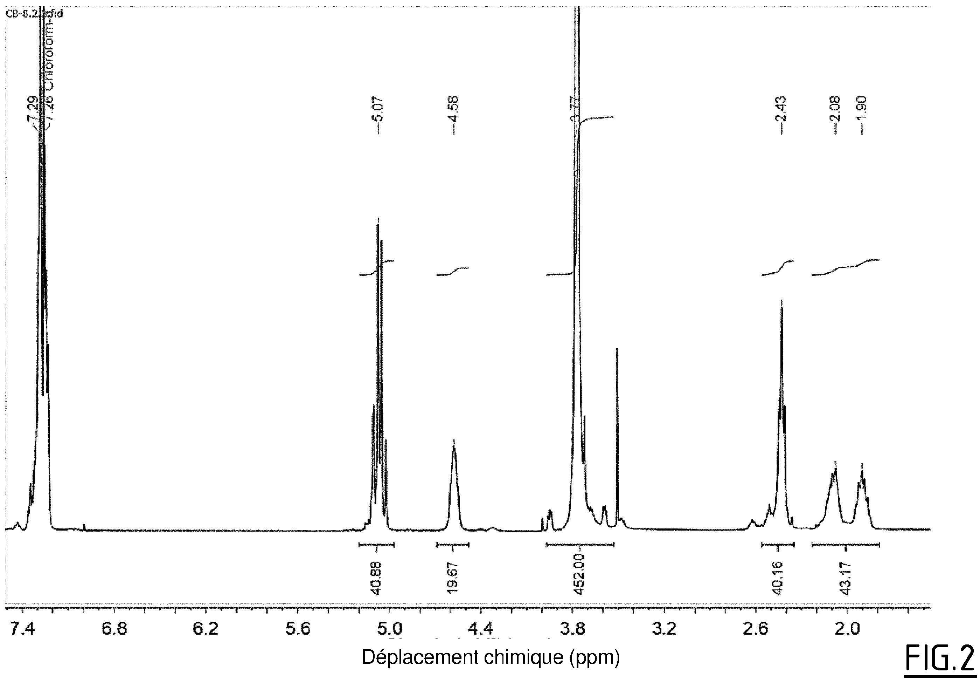

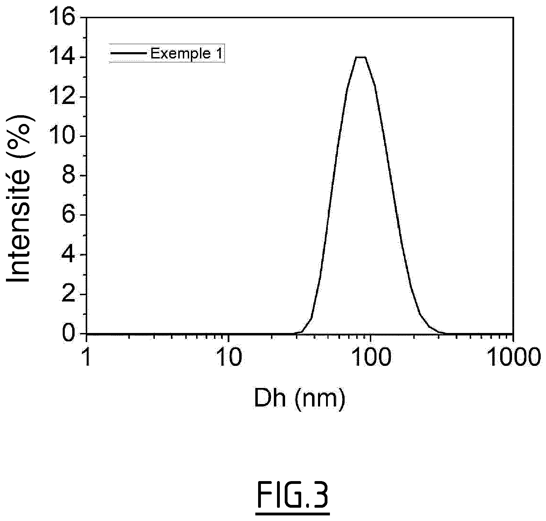

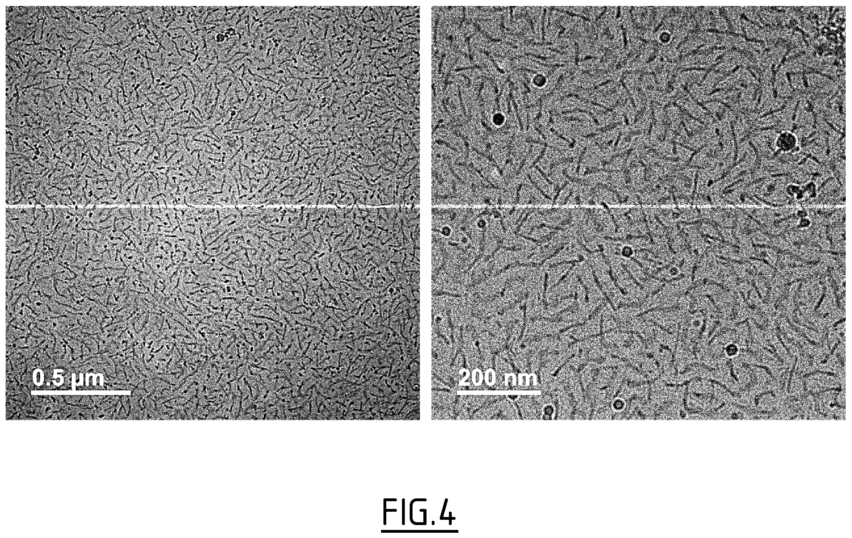

Le monomère NCA du γ-benzyl-*L*-glutamate (300 mg, 1,14 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,05 M contenant le macroamorceur PEG₅ₖ-NH₂ (8 mL, 300 mg, 0,06 mmol) est préparée puis mis à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16h. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche avec un rendement de 85 ± 3% (Figures 1 à 4).

### Exemple 2 : Synthèse concomitante et en l'absence de sels d'un copolymère diblocs peptidique amphiphile poly(éthylène glycol)₅ₖ-block-poly(γ-benzyl-L-glutamate) et de leur nanoparticules correspondantes

Le monomère γ-Benzyl-*L*-glutamate *N*-carboxyanhydride (BLG-NCA) est un réactif chimique commercial qui est distribué par l'entreprise ISOCHEM. Le PEG₅ₖ-NH₂ (Mp = 5516 Da, Ð = 1.02) est distribué par l'entreprise RAPP Polymer. Les autres réactifs sont distribués par l'entreprise Sigma-Aldrich.

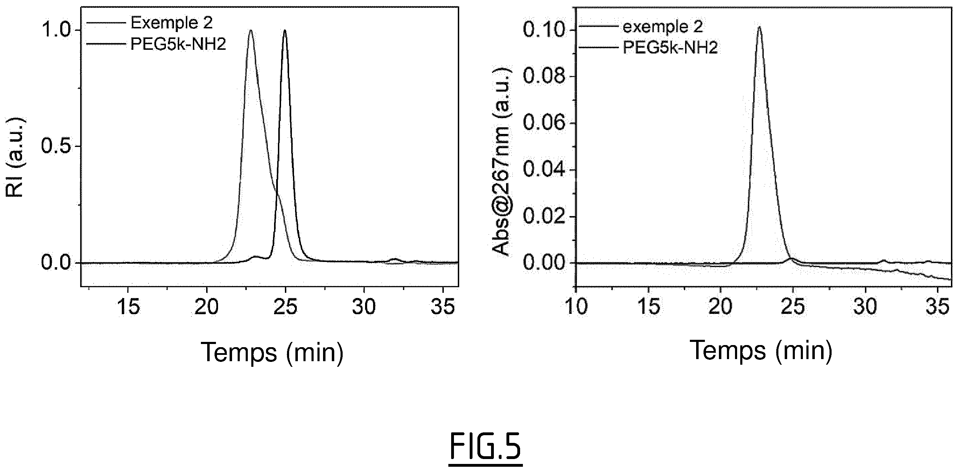

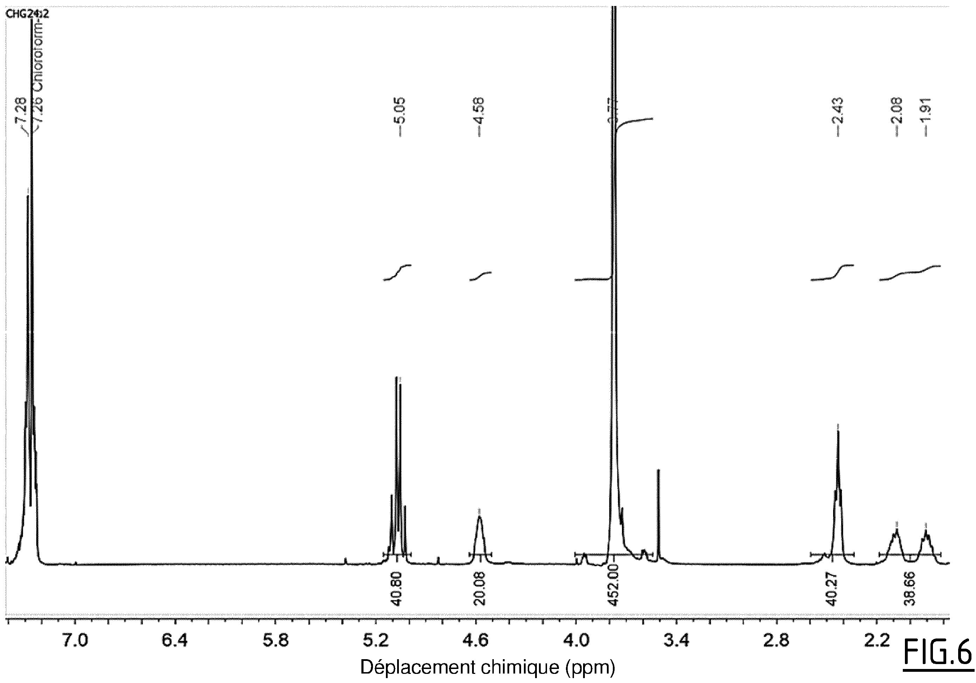

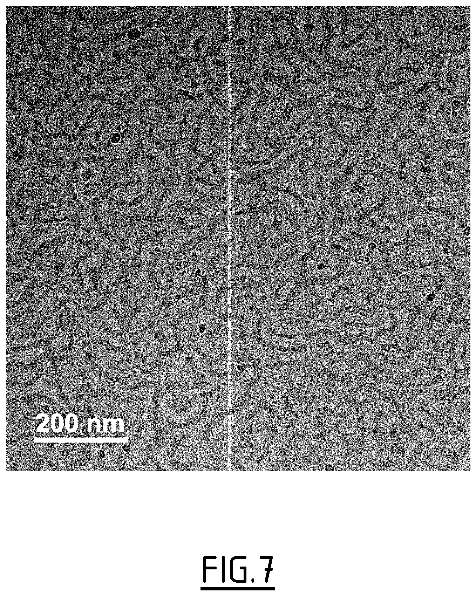

Le monomère NCA du γ-benzyl-*L*-glutamate (300 mg, 1,14 mmol) est mis sous atmosphère inerte dans un tube de Schlenk contenant un barreau magnétique. Le Schlenk est refroidi dans un bain de glace pendant au moins 10 minutes. Parallèlement, le macroamorceur PEG₅ₖ-NH₂ (8 mL, 300 mg, 0,06 mmol) est dilué dans l'eau ultra-pure puis mis à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16h. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche (Figures 5 à 7).

### Exemple 3 : Synthèse concomitante et à fort taux de solide d'un copolymère diblocs peptidique amphiphile poly(éthylène glycol)₅ₖ-block-poly(γ-benzyl-L-glutamate) et de leur nanoparticules correspondantes

Le monomère γ-Benzyl-*L*-glutamate *N*-carboxyanhydride (BLG-NCA) est un réactif chimique commercial qui est distribué par l'entreprise ISOCHEM. Le PEG₅ₖ-NH₂ (Mp = 5516 Da, Ð = 1.02) est distribué par l'entreprise RAPP Polymer. Les autres réactifs sont distribués par l'entreprise Sigma-Aldrich.

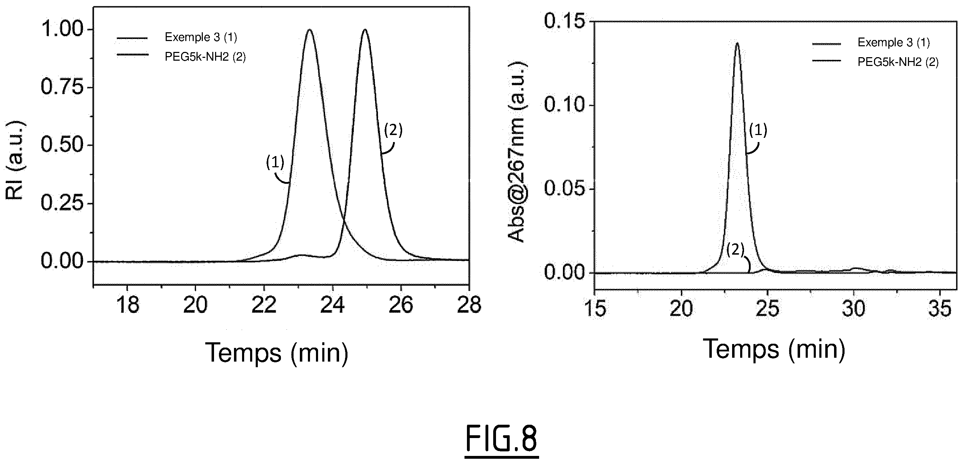

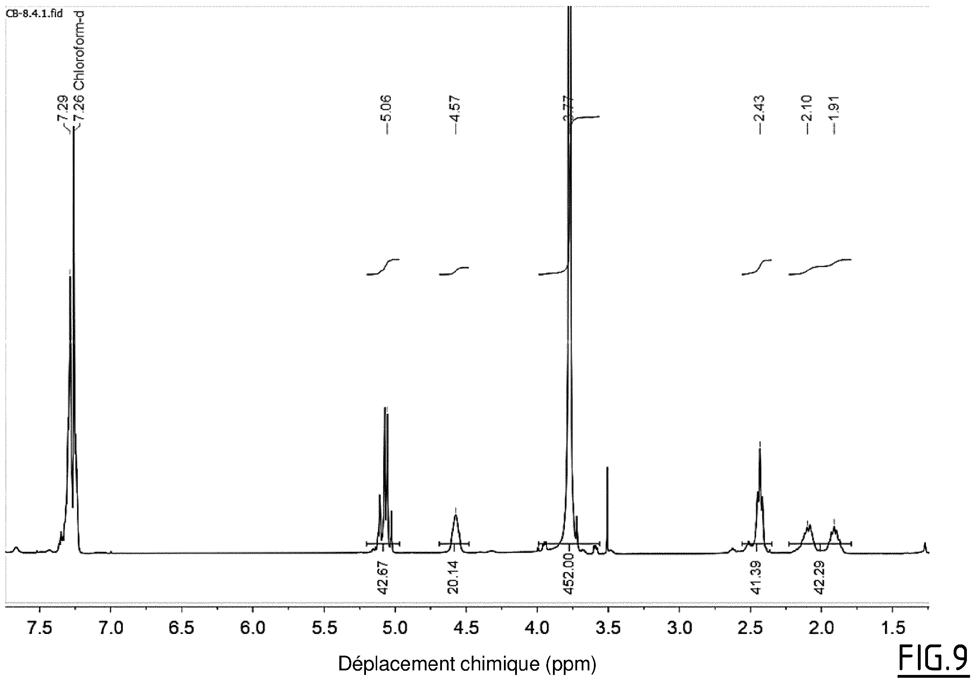

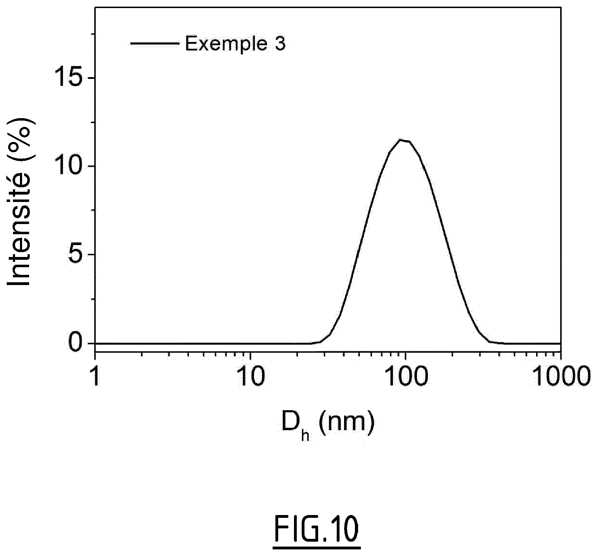

Le monomère NCA du γ-benzyl-*L*-glutamate (600 mg, 2,28 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,05 M contenant le macroamorceur PEG₅ₖ-NH₂ (8 mL, 600 mg, 0,12 mmol) est préparée puis mis à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16h. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche avec un rendement de 77% (Figures 8 à 10).

### Exemple 4 : Procédure d'extension de chaîne d'un copolymère diblocs peptidique amphiphile poly(éthylène glycol)₅ₖ-block-poly(γ-benzyl-L-glutamate)

Le monomère γ-Benzyl-*L*-glutamate *N*-carboxyanhydride (BLG-NCA) est un réactif chimique commercial qui est distribué par l'entreprise ISOCHEM. Le PEG₅ₖ-NH₂ (Mp = 5516 Da, Ð = 1.02) est distribué par l'entreprise RAPP Polymer. Les autres réactifs sont distribués par l'entreprise Sigma-Aldrich.

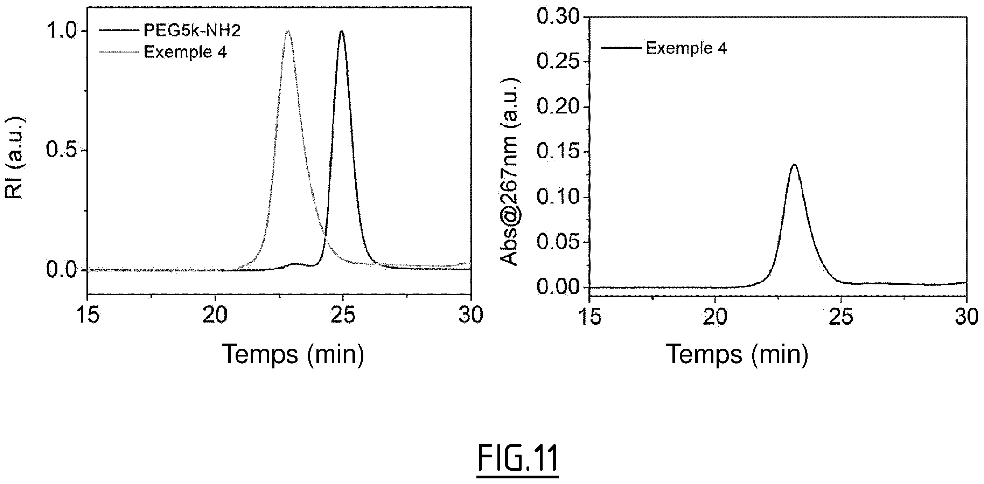

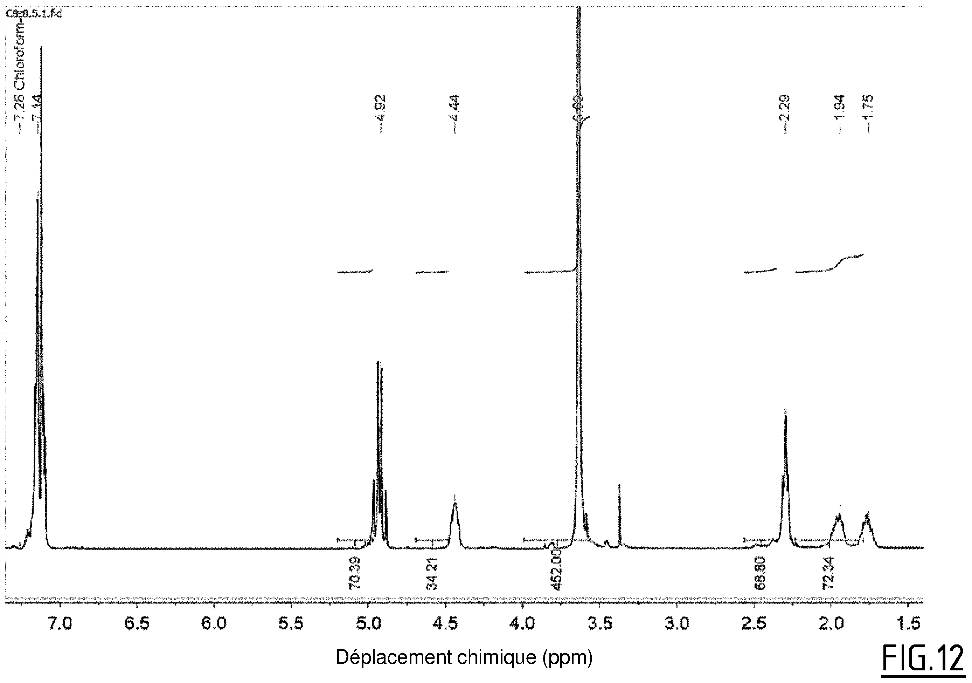

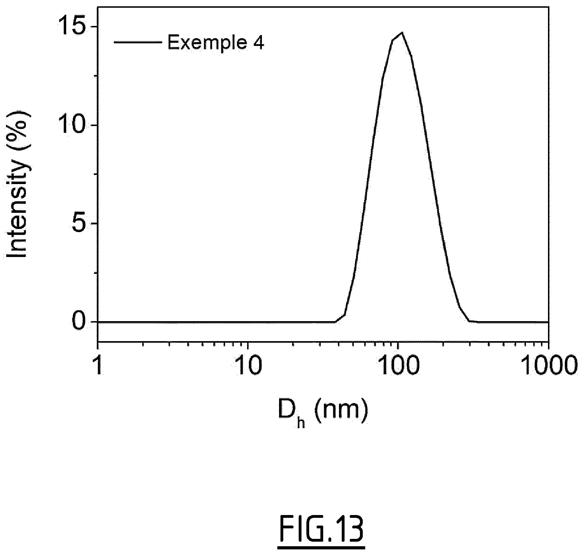

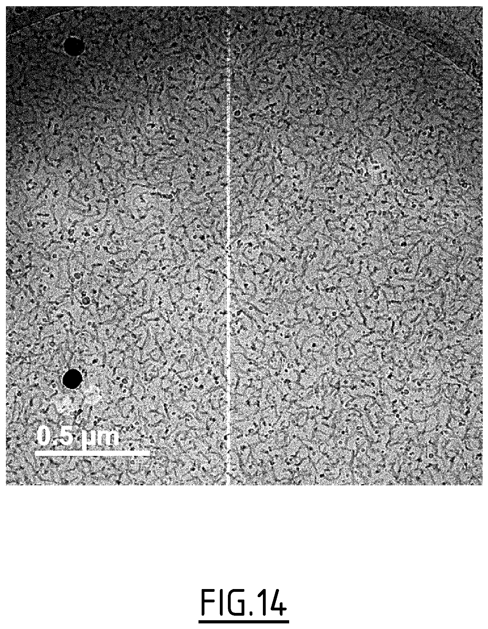

Le monomère NCA du γ-benzyl-*L*-glutamate (300 mg, 1,14 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,05 M contenant le macroamorceur PEG₅ₖ-NH₂ (8 mL, 300 mg, 0,06 mmol) est préparée puis mis à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. Après 90 min, un second ajout de NCA γ-benzyl-*L*-glutamate (300 mg, 1,14 mmol) est réalisé. La réaction est laissée sous agitation 1) d'abord dans un bain d'eau glacée pendant environ 2 heures, 2) puis à température ambiante pendant 16h. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche avec un rendement de 85% (Figures 11 à 14).

### Exemple 5 : Synthèse concomitante d'un copolymère diblocs peptidique amphiphile poly(éthylène glycol)₂ₖ-block-poly(γ-benzyl-L-glutamate) et de leurs nanoparticules correspondantes

Le monomère γ-Benzyl-*L*-glutamate *N*-carboxyanhydride (BLG-NCA) est un réactif chimique commercial qui est distribué par l'entreprise ISOCHEM. Le PEG₂ₖ-NH₂ (Mp = 2022 Da, Ð = 1.04) est distribué par l'entreprise RAPP Polymer. Les autres réactifs sont distribués par l'entreprise Sigma-Aldrich.

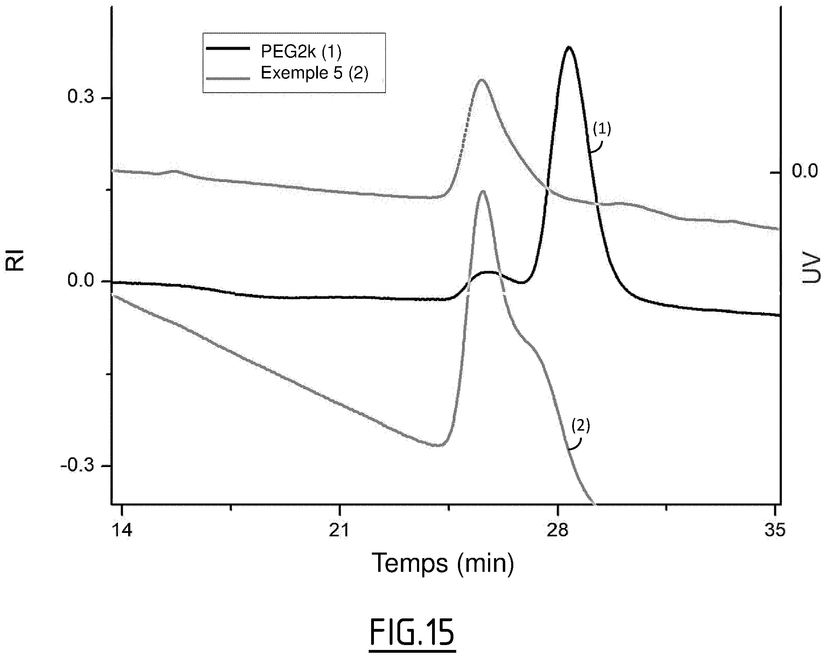

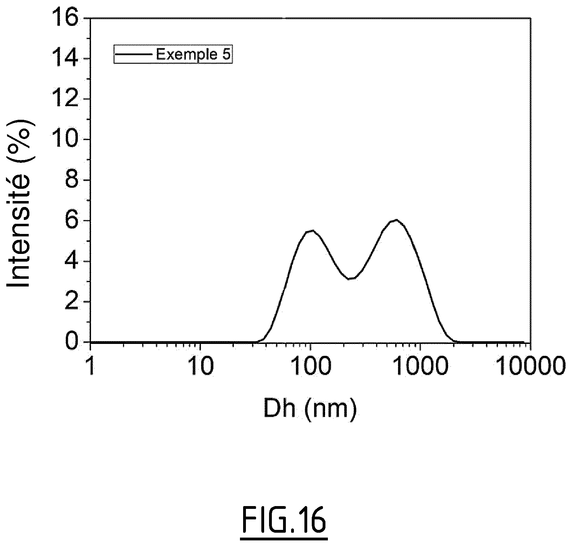

Le monomère NCA du γ-benzyl-*L*-glutamate (30 mg, 0,11 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,2 M contenant le macroamorceur PEG₂ₖ-NH₂ (800 µL, 30 mg, 0,015 mmol) est préparée puis mis à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16h. La dispersion laiteuse évolue en solution aqueuse colloïdale qui est ensuite transférée dans un boudin de dialyse (membrane de dialyse de 3,5 kDa) et dialysée contre de l'eau ultrapure pendant 2 jours. Après lyophilisation, on obtient une poudre blanche (Figures 15 et 16).

### Exemple 6 : Synthèse concomitante d'un copolymère diblocs peptidique amphiphile poly(éthylène glycol)₁₀ₖ-block-poly(γ-benzyl-L-glutamate) et de leur nanoparticules correspondantes

Le monomère γ-Benzyl-*L*-glutamate *N*-carboxyanhydride (BLG-NCA) est un réactif chimique commercial qui est distribué par l'entreprise ISOCHEM. Le PEG₁₀ₖ-NH₂ (Mp = 11153 Da, Ð = 1.05) est distribué par l'entreprise RAPP Polymer. Les autres réactifs sont distribués par l'entreprise Sigma-Aldrich.

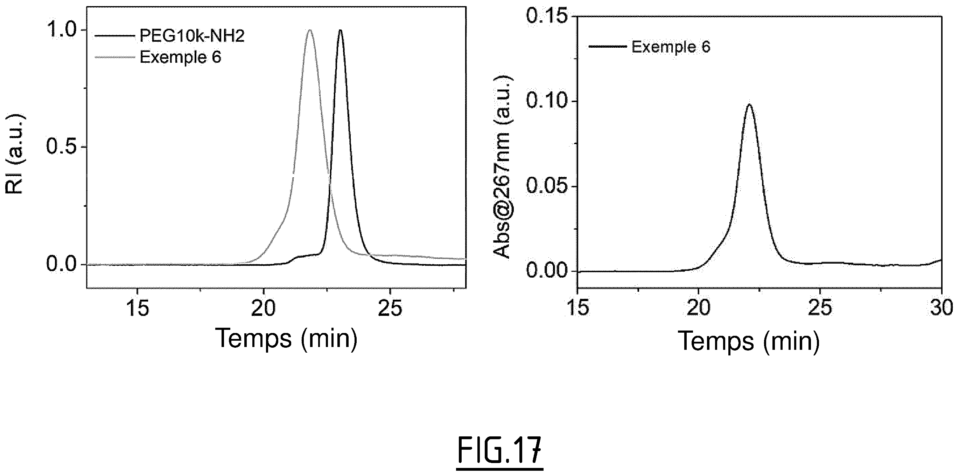

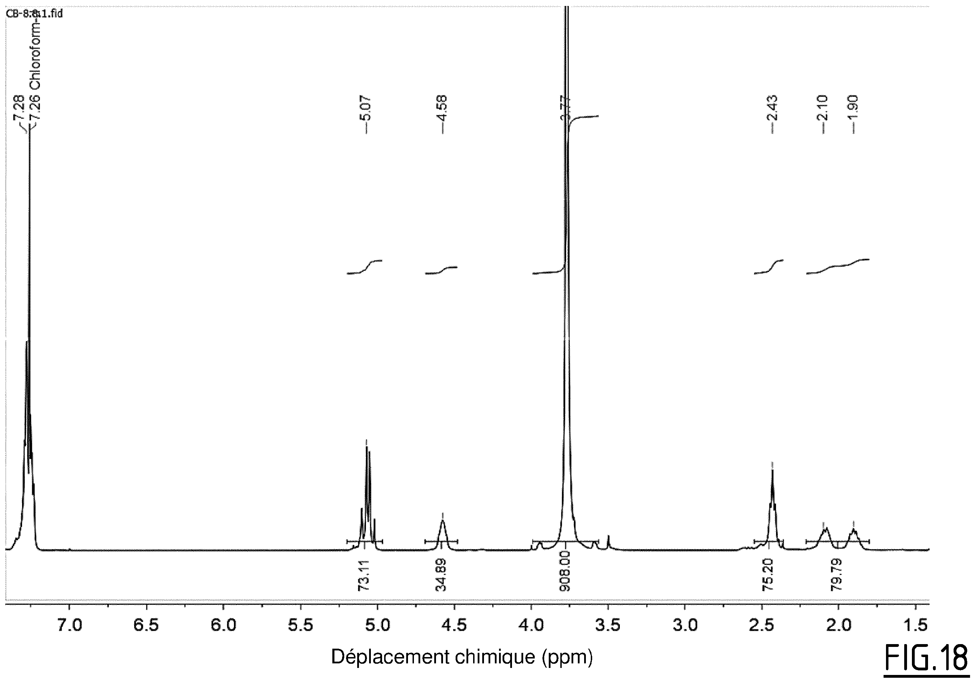

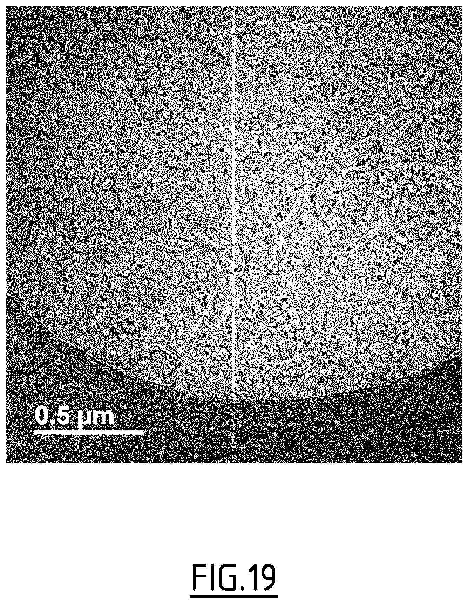

Le monomère NCA du γ-benzyl-*L*-glutamate (300 mg, 1,14 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,05 M contenant le macroamorceur PEG₁₀ₖ-NH₂ (8 mL, 300 mg, 0,03 mmol) est préparée puis mis à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16h. La dispersion laiteuse évolue en un gel qui est ensuite transférée dans un boudin de dialyse (membrane de dialyse de 3,5 kDa) et dialysée contre de l'eau ultrapure pendant 2 jours. Après lyophilisation, on obtient une poudre blanche (Figures 17 à 19).

### Exemple 7 : Synthèse concomitante d'un copolymère diblocs peptidique amphiphile (Elastin-like polypeptide)-block-poly(γ-benzyl-L-glutamate) et de leur nanoparticules correspondantes

Le monomère γ-Benzyl-*L*-glutamate *N*-carboxyanhydride (BLG-NCA) est un réactif chimique commercial qui est distribué par l'entreprise ISOCHEM. L'elastin-like polypeptide (ELP) est une protéine recombinante produite dans la bactérie *E. Coli* au Laboratoire de Chimie des Polymères Organiques de Bordeaux, France. L'ELP utilisé possède une amine primaire à son extrémité N-terminale. Il est de structure MW (VPGVP VPGMG VPGVG VPGVG)₁₀ et d'une masse molaire de 17 035 Da. Les autres réactifs non classiques sont distribués par l'entreprise Sigma-Aldrich.

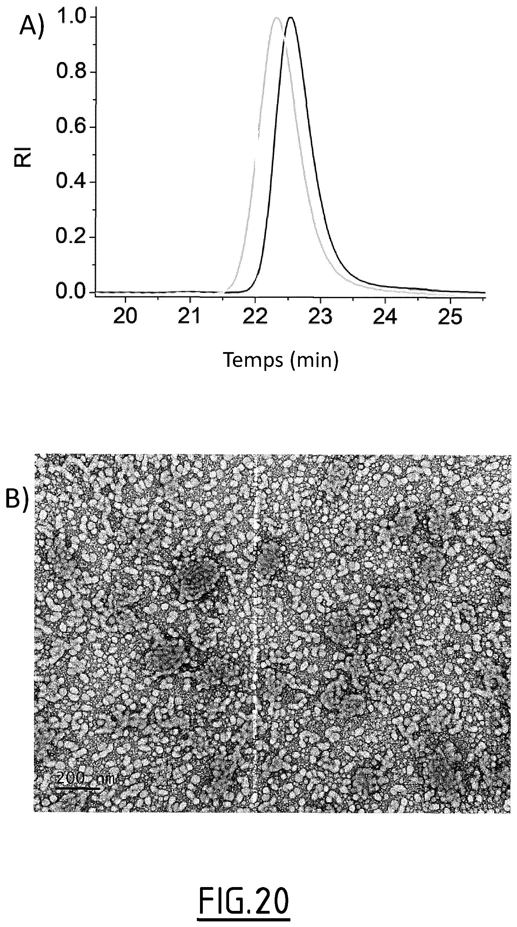

Le monomère NCA du γ-benzyl-*L*-glutamate (10 mg, 0.04 mmol) est mis sous atmosphère inerte dans un tube à essai contenant un barreau magnétique. Le tube est refroidi dans un bain de glace pendant au moins 10 minutes. Parallèlement, une solution aqueuse de NaHCO₃ 0.05 M contenant le macroamorceur ELP (2.7 mL, 10 mg, 0,001 mmol) est préparée puis mis à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation magnétique à 4°C durant 20h. La dispersion laiteuse évolue en une dispersion turbide à température ambiante qui est ensuite transférée dans un boudin de dialyse (membrane de dialyse de 3,5 kDa) et dialysée contre de l'eau ultrapure pendant 2 jours à 4°C. Après lyophilisation, une poudre blanche est obtenue (Figure 20).

### Exemple 8 : Synthèse concomitante d'un copolymère diblocs peptidique amphiphile poly(éthylène glycol)-block-poly(ε-Boc-L-Lysine) et de leurs nanoparticules correspondantes

Le monomère de ε-tert-butyloxycarbonyl-*L*-lysine *N*-carboxyanhydride (LysBOC-NCA) est un produit commercial distribué par l'entreprise ISOCHEM. Le PEG₅ₖ-NH₂ (Mp = 5516 Da, Ð = 1.02) est distribué par l'entreprise RAPP Polymer. Les autres réactifs non-classiques sont distribués par l'entreprise Sigma-Aldrich.

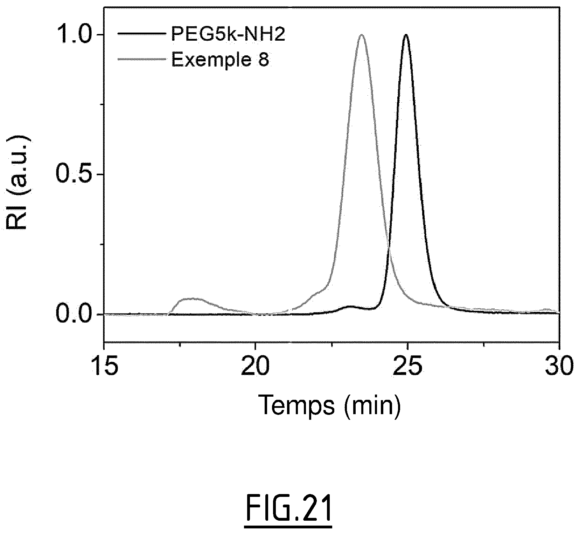

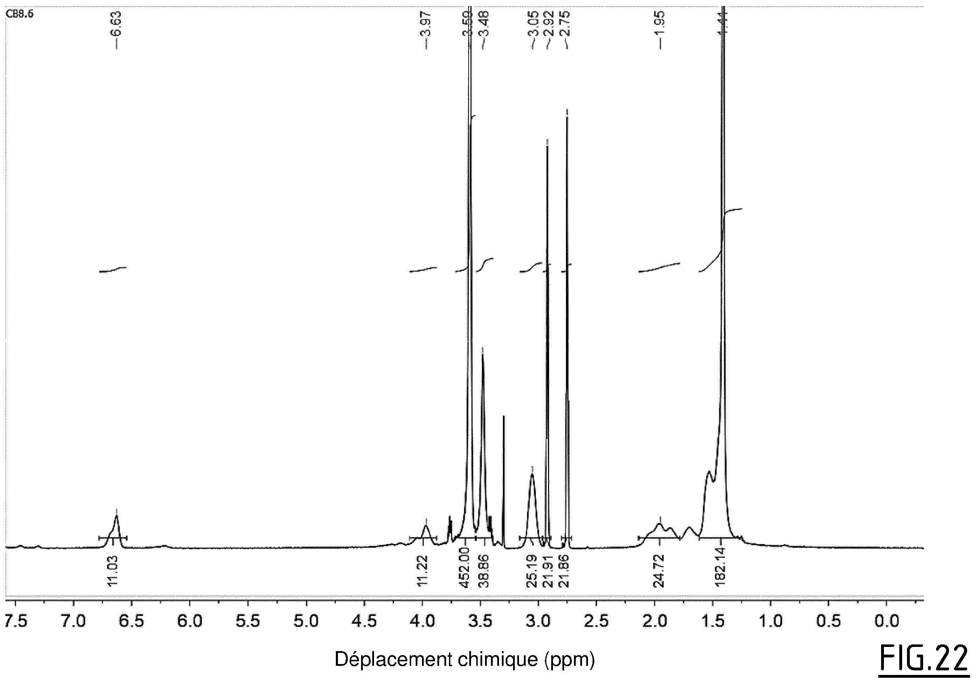

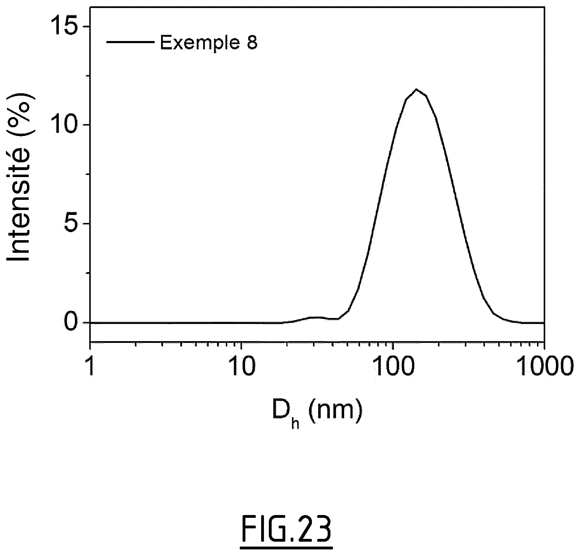

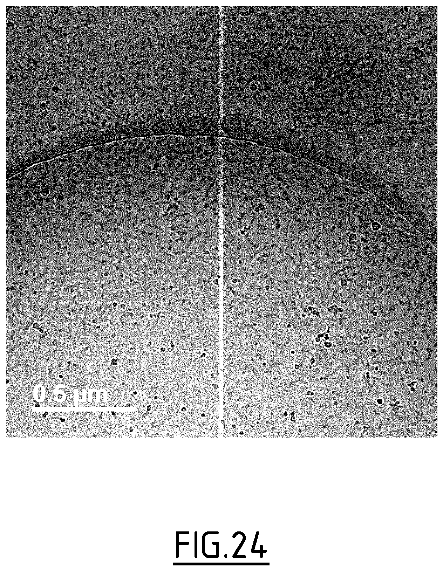

Le monomère NCA du ε-Boc-*L*-Lysine (310 mg, 1,14 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,05 M contenant le macroamorceur PEG₅ₖ-NH₂ (8 mL, 300 mg, 0,06 mmol) est préparée puis mis à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16h. La dispersion laiteuse évolue en solution aqueuse colloïdale opalescente qui est ensuite transférée dans un boudin de dialyse (membrane de dialyse de 3,5 kD) et dialysée contre de l'eau ultrapure pendant 2 jours. Après lyophilisation, on obtient une poudre blanche avec un rendement de 79 % (Figures 21 à 24).

### Exemple 9 : Synthèse concomitante d'un copolymère triblocs peptidique amphiphile poly(éthylène glycol)-block-poly(ε-Boc-L-Lysine)-block-poly(γ-benzyl-L-glutamate) et de leurs nanoparticules correspondantes

Les monomères γ-Benzyl-*L*-glutamate *N*-carboxyanhydride (BLG-NCA) et ε-tert-butyloxycarbonyl-*L*-lysine *N*-carboxyanhydride (LysBOC-NCA) sont des produits commerciaux distribués par l'entreprise ISOCHEM. Le PEG₅ₖ-NH₂ (Mp = 5516 Da, Ð = 1.02) est distribué par l'entreprise RAPP Polymer. Les autres réactifs non-classiques sont distribués par l'entreprise Sigma-Aldrich.

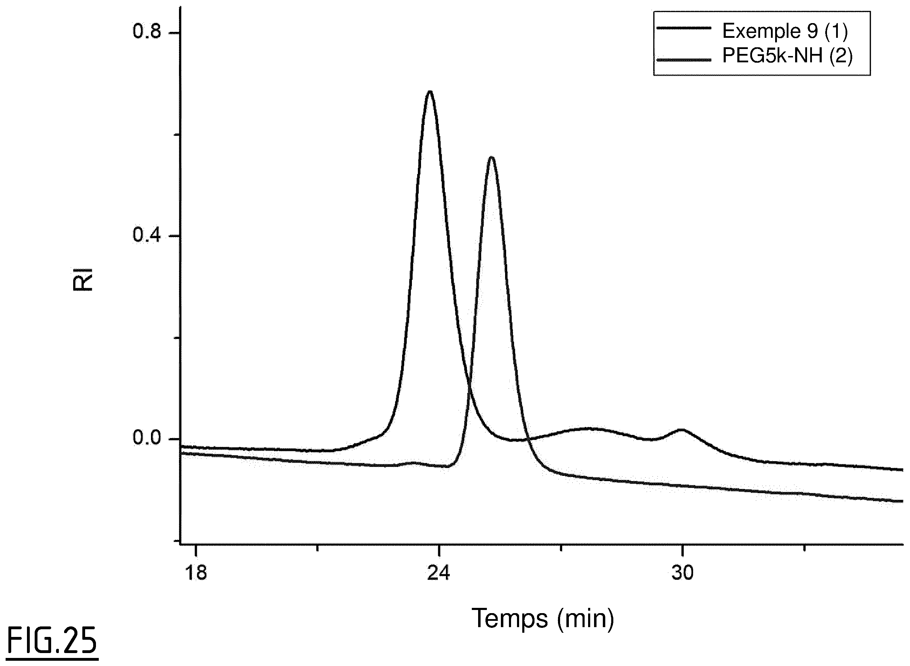

Le monomère NCA du ε-Boc-*L*-Lysine (300 mg, 1,10 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,2 M contenant le macroamorceur PEG₅ₖ-NH₂ (8 mL, 300 mg, 0,06 mmol) est préparée puis mis à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation dans un bain d'eau glacée pendant environ 15 minutes. Parallèlement, le monomère NCA du γ-benzyl-*L*-glutamate (BLG-NCA, 300 mg, 1,14 mmol) est mis sous atmosphère inerte dans un tube de Schlenk contenant un barreau magnétique. Le Schlenk est refroidi dans un bain de glace pendant au moins 10 minutes. En restant dans ce bain de glace, la solution aqueuse colloïdale opalescente est mélangée à la poudre de BLG-NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16h. La dispersion laiteuse évolue en solution aqueuse colloïdale opalescente qui est ensuite transférée dans un boudin de dialyse (membrane de dialyse de 3,5 kD) et dialysée contre de l'eau ultrapure pendant 2 jours. Après lyophilisation, on obtient une poudre blanche avec un rendement de 75 ± 3% (Figure 25).

### Exemple 10 : Synthèse concomitante d'un copolymère diblocs peptidique amphiphile poly(éthylène glycol)₅ₖ-block-poly(L-leucine) et de leur nanoparticules correspondantes

Le monomère *L*-leucine *N*-carboxyanhydride (LEU-NCA) est un réactif chimique commercial qui est distribué par l'entreprise ISOCHEM. Le PEG₅ₖ-NH₂ (Mp = 5516 Da, Ð = 1.02) est distribué par l'entreprise RAPP Polymer. Les autres réactifs sont distribués par l'entreprise Sigma-Aldrich.

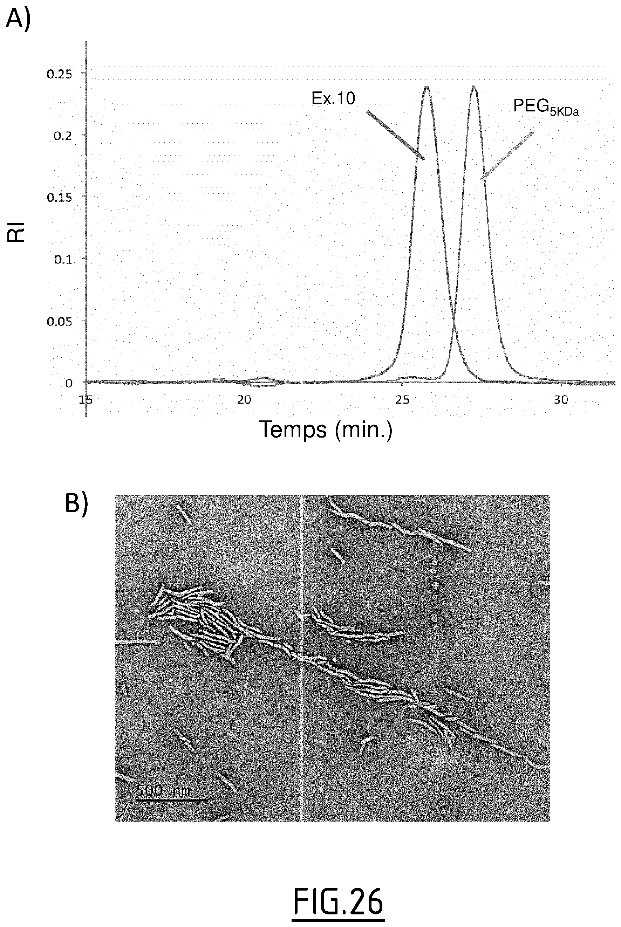

Le monomère NCA de la leucine (300 mg, 1,9 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,05 M contenant le macroamorceur PEG₅ₖ-NH₂ (8 mL, 300 mg, 0,06 mmol) est préparée puis mise à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16 heures. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche avec un rendement de 77 % (Figure 26).

### Exemple 11 : Synthèse concomitante d'un copolymère diblocs peptidique amphiphile poly(éthylène glycol)₅ₖ-block-poly(L-phenylalanine) et de leur nanoparticules correspondantes

Le monomère *L*-phenylalanine *N*-carboxyanhydride (PHE-NCA) est un réactif chimique commercial qui est distribué par l'entreprise ISOCHEM. Le PEG₅ₖ-NH₂ (Mp = 5516 Da, Ð = 1.02) est distribué par l'entreprise RAPP Polymer. Les autres réactifs sont distribués par l'entreprise Sigma-Aldrich.

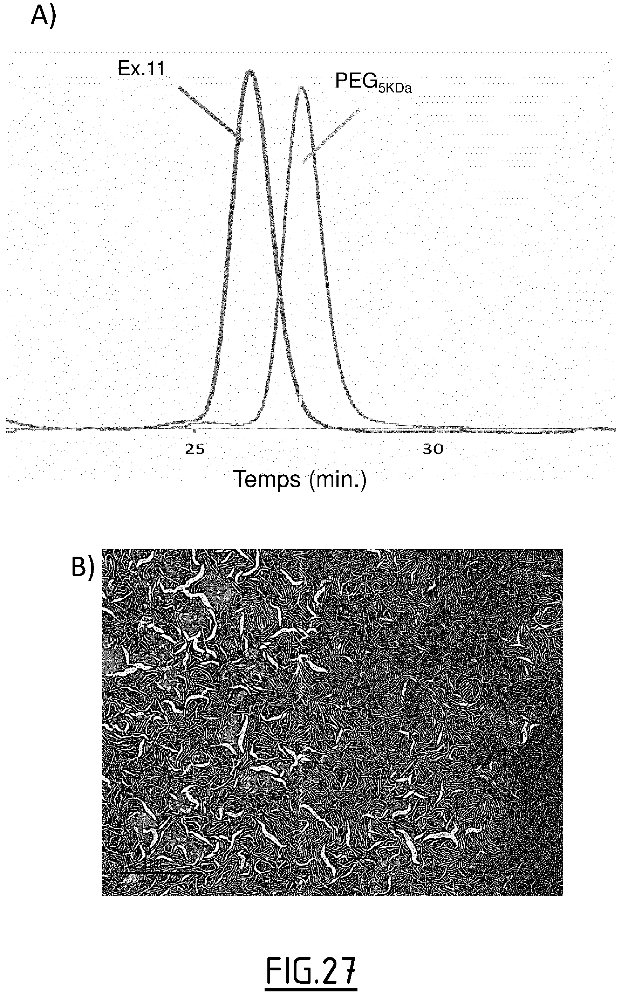

Le monomère NCA de la phénylalanine (150 mg, 0.8 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,05 M contenant le macroamorceur PEG₅ₖ-NH₂ (8 mL, 300 mg, 0,06 mmol) est préparée puis mise à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16 heures. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche avec un rendement de 72 % (Figure 27).

### Exemple 12 : Synthèse concomitante d'un copolymère triblocs peptidique amphiphile poly(γ-benzyl-L-glutamate)-block-poly(éthylène glycol)₅ₖ-block-poly(γ-benzyl-L-glutamate) et de leur nanoparticules correspondantes

Le monomère γ-Benzyl-*L*-glutamate *N*-carboxyanhydride (BLG-NCA) est un réactif chimique commercial qui est distribué par l'entreprise ISOCHEM. Le NH₂-PEG₅ₖ-NH₂ (PEG-2arm) est distribué par l'entreprise RAPP Polymer. Les autres réactifs sont distribués par l'entreprise Sigma-Aldrich.

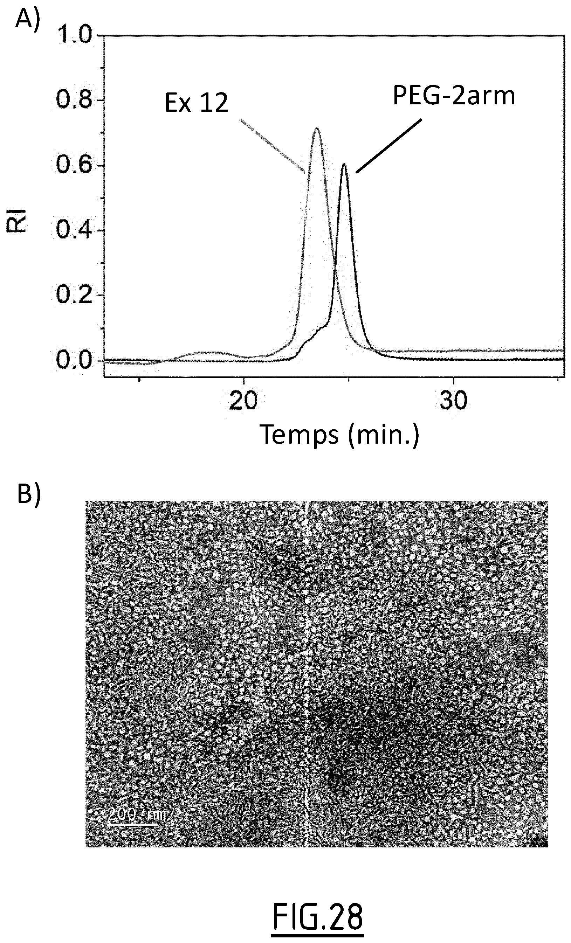

Le monomère NCA de γ-Benzyl-*L*-glutamate *N*-carboxyanhydride (BLG-NCA) (29 mg, 0.11 mmol) est mis sous atmosphère inerte dans un tube à essaicontenant un barreau magnétique. Le tube est refroidi dans un bain de glace pendant au moins 10 minutes. Parallèlement, une solution aqueuse de NaHCO₃ 0,05 M contenant le macroamorceur NH₂-PEG₅ₖ-NH₂ (0.85 mL, 35 mg) est préparée puis mise à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16 heures. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche (Figure 28).

### Exemple 13 : Synthèse concomitante d'un copolymère en étoile peptidique amphiphile poly(γ-benzyl-L-glutamate)₄-block-poly(éthylène glycol)₅ₖ et de leur nanoparticules correspondantes

Le monomère γ-Benzyl-*L*-glutamate *N*-carboxyanhydride (BLG-NCA) est un réactif chimique commercial qui est distribué par l'entreprise ISOCHEM. Le PEG. 4arm est distribué par l'entreprise RAPP Polymer (en étoile: 4 extrémité NH₂). Les autres réactifs sont distribués par l'entreprise Sigma-Aldrich.

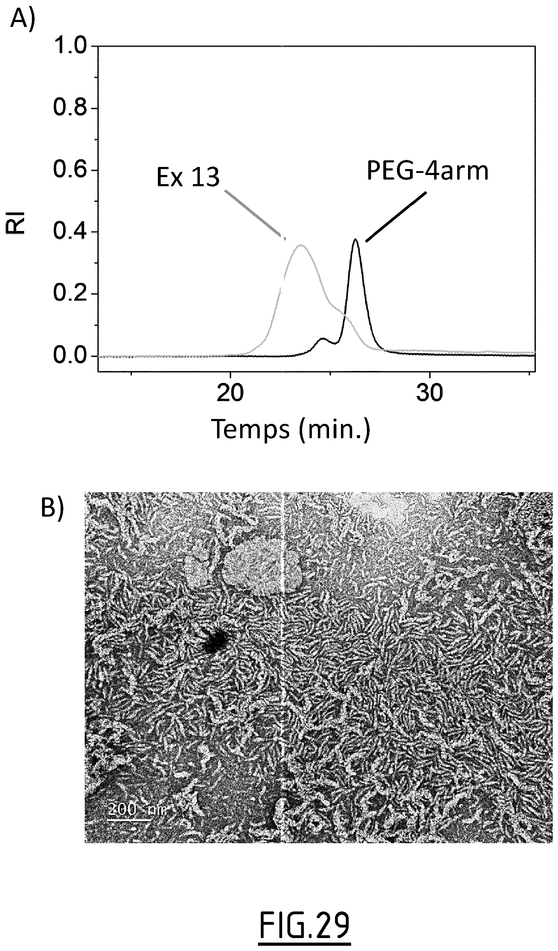

Le monomère NCA de γ-Benzyl*-L*-glutamate *N*-carboxyanhydride (BLG-NCA) (18 mg, 0.07 mmol) est mis sous atmosphère inerte dans un tube à essaicontenant un barreau magnétique. Le tube est refroidi dans un bain de glace pendant au moins 10 minutes. Parallèlement, une solution aqueuse de NaHCO₃ 0,05 M contenant le macroamorceur PEG₅ₖ-4arm (0.70 mL, 35 mg) est préparée puis mise à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16 heures. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche (Figure 29).

### Exemple 14 : Synthèse concomitante d'un copolymère diblocs peptidique amphiphile poly(sarcosine)-block-poly(γ-benzyl-L-glutamate) et de leur nanoparticules correspondantes

Le monomère γ-Benzyl-*L*-glutamate *N*-carboxyanhydride (BLG-NCA) est un réactif chimique commercial qui est distribué par l'entreprise ISOCHEM. La poly(sarcosine) PSar (Mp = 2100 Da, Ð = 1.03) est synthétisée par polymérisation par ouverture de cycle conventionnelle au laboratoire. Les autres réactifs sont distribués par l'entreprise Sigma-Aldrich.

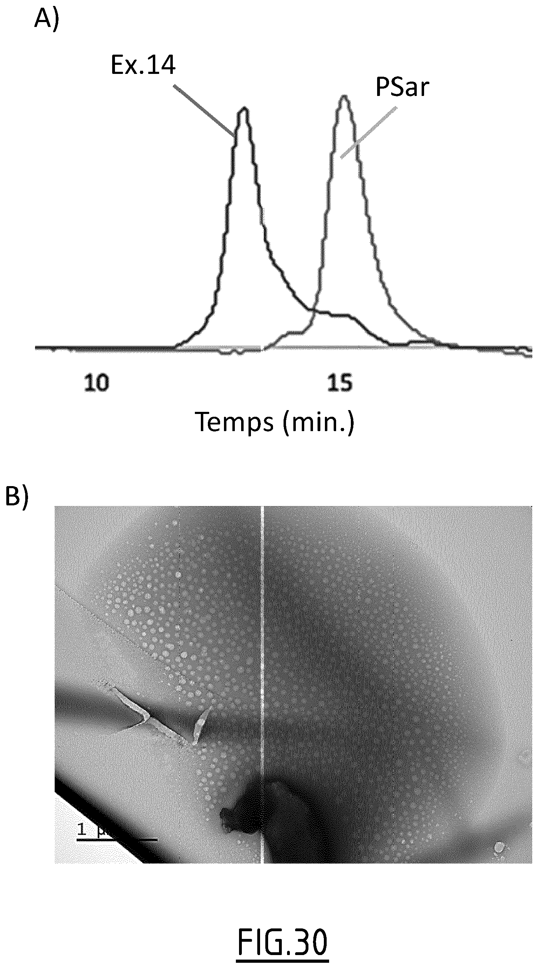

Le monomère NCA du γ-benzyl-*L*-glutamate (120 mg, 0,46 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,05 M contenant le macroamorceur (4 mL, 50 mg, 0,02 mmol) est préparée puis mise à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16 heures. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche avec un rendement de 55% (Figures 30).

### Exemple 15 : Synthèse concomitante d'un copolymère diblocs peptidique amphiphile d'acide poly(L-glutamique)-block-poly(γ-benzyl-L-glutamate) et de leur nanoparticules correspondantes

Le monomère γ-Benzyl-*L*-glutamate *N*-carboxyanhydride (BLG-NCA) est un réactif chimique commercial qui est distribué par l'entreprise ISOCHEM. L'acide poly(L-glutamique) PGA (Mw = 6600 g/mol) est synthétisé par polymérisation par ouverture de cycle conventionnelle au laboratoire. Les autres réactifs sont distribués par l'entreprise Sigma-Aldrich.

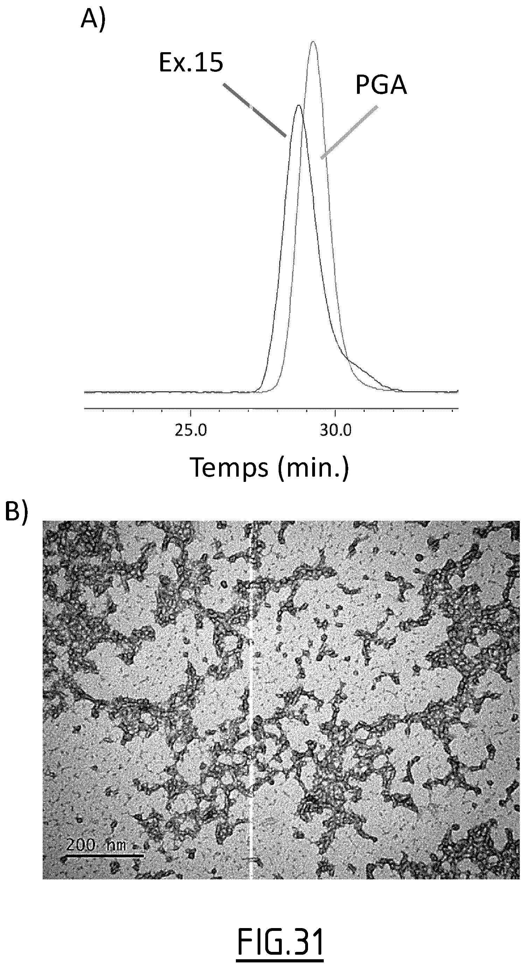

Le monomère NCA du γ-benzyl-*L*-glutamate (300 mg, 1,14 mmol) est mis sous atmosphère inerte dans un tube de Schlenk contenant un barreau magnétique. Le Schlenk est refroidi dans un bain de glace pendant au moins 10 minutes. Parallèlement, une solution aqueuse de NaHCO₃ 0,20 M contenant le macroamorceur (8 mL, 300 mg, 0,45 mmol) est préparée puis mise à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16 heures. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours (cliché TEM, figure 31). Après lyophilisation, pour pouvoir analyser le copolymère, on déprotège le bloc PBLG dans des conditions acides douces (MSA, TFA) pour obtenir une poudre blanche que l'on peut analyser en SEC aqueuse (tampon juanito) (Figure 31).

### Exemple 16 : Synthèse concomitante d'un copolymère diblocs peptidique amphiphile polysaccharide-block-poly(γ-benzyl-L-glutamate) et de leur nanoparticules correspondantes

Le monomère γ-Benzyl-*L*-glutamate *N*-carboxyanhydride (BLG-NCA) est un réactif chimique commercial qui est distribué par l'entreprise ISOCHEM. Le polyaminosaccharide (Polysacc., Mw = 7750 g/mol) est synthétisé par polymérisation anionique de monomères β-lactame au laboratoire. Les autres réactifs sont distribués par l'entreprise Sigma-Aldrich.

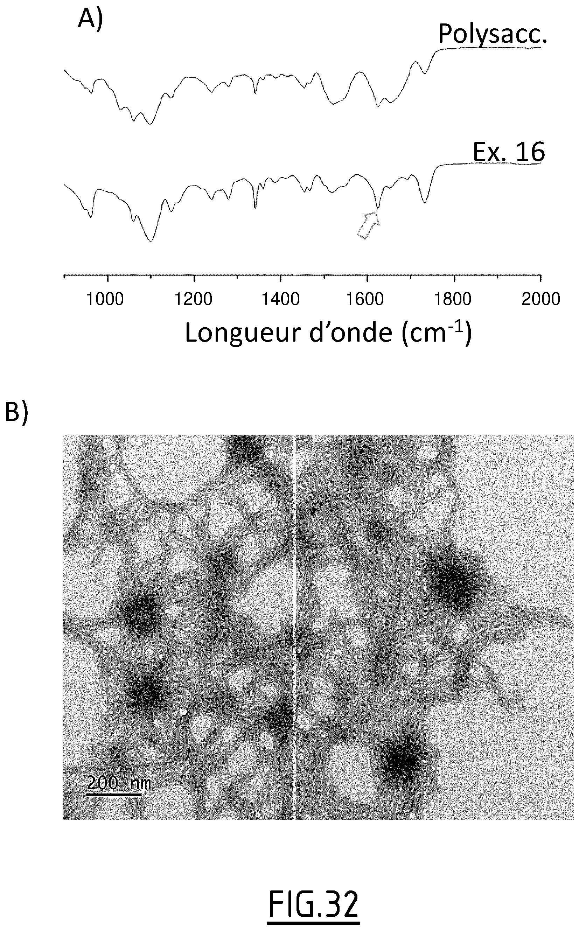

Le monomère NCA du γ-benzyl-*L*-glutamate (13 mg, 0.05 mmol) est mis sous atmosphère inerte dans un tube à essai contenant un barreau magnétique. Le tube est refroidi dans un bain de glace pendant au moins 10 minutes. Parallèlement, une solution aqueuse de NaHCO₃ 0,05 M contenant le macroamorceur (2 mL, 20 mg, 0,003 mmol) est préparée puis mise à refroidir dans un bain de glace pendant au moins 10 minutes. En restant dans le bain de glace, la solution aqueuse est ajoutée à la poudre de NCA sous agitation forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16 heures. La dispersion laiteuse évolue en solution aqueuse colloïdale blanchâtre qui est ensuite transférée dans un boudin de dialyse (membrane de dialyse de 3,5 kDa) et dialysée contre de l'eau ultrapure pendant 2 jours. Après lyophilisation, on obtient une poudre blanche (Figure 32).

### Exemple 17 : Synthèse concomitante d'un copolymère diblocs peptidique amphiphile poly(éthylène glycol)₅ₖ-block-poly(γ-benzyl-L-glutamate) et de leur nanoparticules correspondantes à très petite échelle

Le monomère γ-Benzyl-*L*-glutamate *N*-carboxyanhydride (BLG-NCA) est un réactif chimique commercial qui est distribué par l'entreprise ISOCHEM. Le PEG₅ₖ-NH₂ (Mp = 5516 Da, Ð = 1.02) est distribué par l'entreprise RAPP Polymer. Les autres réactifs sont distribués par l'entreprise Sigma-Aldrich.

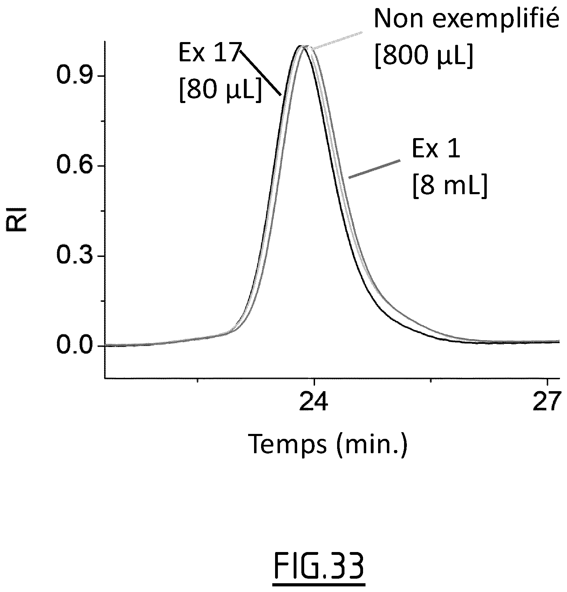

Le monomère NCA du γ-benzyl-*L*-glutamate (3 mg, 0.01 mmol) est mis sous atmosphère inerte dans un eppendorf contenant un petit barreau magnétique, qui est refroidi dans un bain de glace pendant au moins 10 minutes. Parallèlement, une solution aqueuse de NaHCO₃ 0,05 M contenant le macroamorceur PEG₅ₖ-NH₂ (80 µL, 3 mg) est préparée puis mise à refroidir dans un bain de glace pendant au moins 10 minutes. En restant dans le bain de glace, la solution aqueuse est ajoutée à la poudre de NCA sous agitation magnétique forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16 heures. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche (Figure 33).

### Exemple 18 : Synthèse concomitante d'un copolymère diblocs peptidique amphiphile acide désoxyribonucléique-block-poly(γ-benzyl-L-glutamate) et de leur nanoparticules correspondantes à très petite échelle

Le monomère γ-Benzyl-*L*-glutamate *N*-carboxyanhydride (BLG-NCA) est un réactif chimique commercial qui est distribué par l'entreprise ISOCHEM. L'acide désoxyribonucléique DNA (TTT)₁₅ (Mw=4680 g/mol) est distribué par l'entreprise IDT Technologies. Les autres réactifs sont distribués par l'entreprise Sigma-Aldrich.

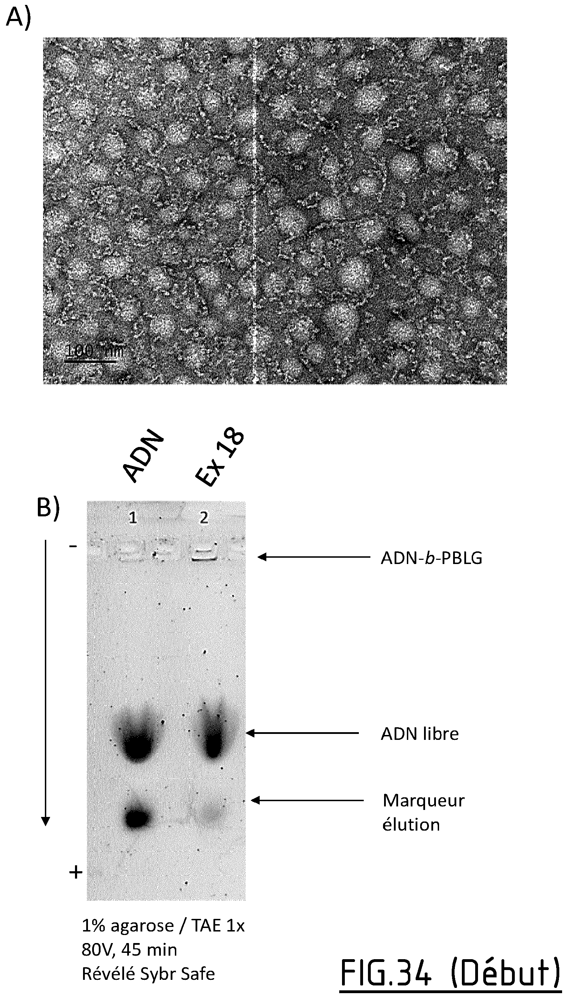

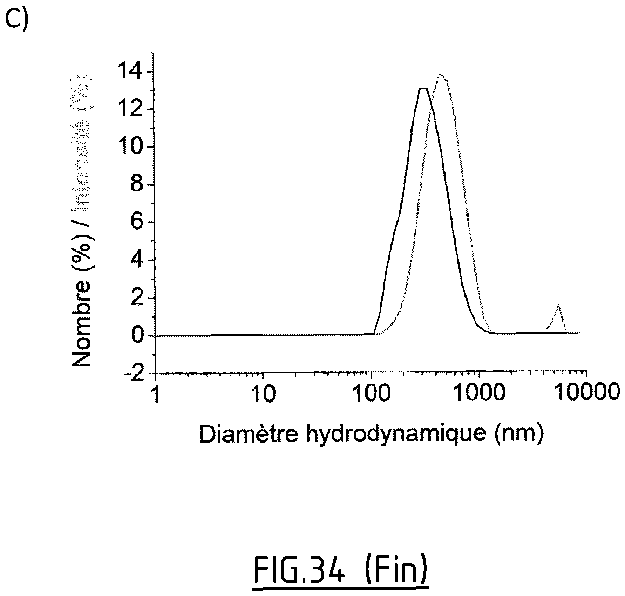

Le monomère NCA du γ-benzyl-*L*-glutamate (5 mg) est mis sous atmosphère inerte dans un eppendorf qui est refroidi dans un bain de glace pendant au moins 10 minutes. Parallèlement, une solution aqueuse de NaHCO₃ 0,05 M contenant le macroamorceur ADN (1 mL, 5 mg) est préparée puis mise à refroidir dans un bain de glace pendant au moins 10 minutes. En restant dans le bain de glace, la solution aqueuse est ajoutée à la poudre de NCA sous agitation magnétique forte. Il en résulte une dispersion laiteuse résultant de la non miscibilité du monomère dans la phase aqueuse. La réaction est laissée sous agitation 1) d'abord dans un bain d'eau glacée pendant environ 2 heures, 2) puis à température ambiante pendant 16 heures. La dispersion laiteuse évolue en solution aqueuse colloïdale opalescente qui est ensuite transférée dans un boudin de dialyse (membrane de dialyse de 3,5 kDa) et dialysée contre de l'eau ultrapure pendant 2 jours. Après lyophilisation, on obtient une poudre blanche (Figure 34).

Les tableaux 1 et 2 ci-après fournissent les caractéristiques moléculaires et physio-chimiques des copolymères synthétisés par ROPISA ainsi que de leurs nanoparticules selon les exemples ci-dessus.

**[Tableau 1]**

| **Copolymère** | **Tampon** | **Initiateur** | **M/I** g/mol | **Tₛ (%)** | **M/I** | ***Mₙ*** g/mol | ***Mₙ*** g/mol | ***Ð*** |
|---|---|---|---|---|---|---|---|---|
| | | | Th. | | ¹H NMR | | SEC* | |
| PEG₅ₖ-NH₂ | - | - | - | - | - | - | 6996 | 1.02 |
| Ex.1 | NaHCO₃ 50mM | PEG₅ₖ-NH₂ | 19 | 7 | 20 | 9380 | 11940 | 1.05 |
| Ex.2 | MQ | PEG₅ₖ-NH₂ | 19 | 7 | 20 | 9380 | 13470 | 1.14 |
| Ex.3 | NaHCO₃ 50mM | PEG₅ₖ-NH₂ | 19 | 13 | 21 | 9600 | 12250 | 1.06 |
| Ex.4 | NaHCO₃ 50mM | PEG₅ₖ-NH₂ | 38 | 10 | 35 | 12670 | 14370 | 1.09 |
| Ex.5 | NaHCO₃ 200mM | PEG₂ₖ-NH₂ | 8 | 13 | - | - | 4067 | 1.20 |
| Ex.6 | NaHCO₃ 50mM | PEG₁₀ₖ-NH₂ | 38 | 7 | 37 | 18100 | 24660 | 1.12 |
| Ex.7 | NaHCO₃ 50mM | ELPM40 | 65 | 0.7 | 30 | 23600 | 27000 | 1.03 |
| Ex.8 | NaHCO₃ 50mM | PEG₅ₖ-NH₂ | 19 | 7 | 21 | 9790 | 11750 | 1.09 |
| Ex.9 | NaHCO₃ 200mM | PEG₅ₖ-NH₂ | 38 | 13 | - | - | 12220 | 1.05 |
| Ex.10 | NaHCO₃ 50mM | PEG₅ₖ-NH₂ | 32 | 7 | - | - | 10600 | 1.04 |
| Ex.11 | NaHCO₃ 50mM | PEG₅ₖ-NH₂ 2arms- | 13 | 5 | - | - | - | - |
| Ex.12 | NaHCO₃ 50mM | PEG₅ₖ-NH₂ 4arms- | 19 | 8 | 18 | 9900 | 14700 | 1.10 |
| Ex.13 | NaHCO₃ 50mM | PEG₅ₖ-NH₂ | 10 | 8 | 16 | 8500 | 11700 | 1.32 |
| Ex.14 | NaHCO₃ 50mM | PSar | 19 | 7 | - | - | 7100 | 1.34 |
| Ex.15 | NaHCO₃ 50mM | PGA | | | | | | |
| Ex.16 | NaHCO₃ 50mM | Polysacc. | 19 | 1.6 | - | - | - | - |
| Ex.17 | NaHCO₃ 50mM | PEG₅ₖ-NH₂ | 19 | 7 | 20 | 9380 | 11900 | 1.05 |
| Ex.18 | NaHCO₃ 50mM | DNA | 19 | 5 | - | - | - | - |

| | | | | | | | | |
|---|---|---|---|---|---|---|---|---|
| * Masse moléculaire moyenne en nombre (***M*ₙ**) et dispersité (***Ð***) déterminées par SEC dans du DMF (+LiBr) en utilisant une courbe de calibration au polystyrène. | | | | | | | | |

**[Tableau 2]**

| **Copolymère** | **Tampon** | **Initiateur** | ***Dₕ* (σ)** nm | **Rendement** % |
|---|---|---|---|---|
| | | | DLS | |
| PEG₅ₖ-NH₂ | - | - | - | - |
| Ex.1 | NaHCO₃ 50mM | PEG₅ₖ-NH₂ | 79 (0.08) | 87 |
| Ex.2 | MQ | PEG₅ₖ-NH₂ | - | - |
| Ex.3 | NaHCO₃ 50mM | PEG₅ₖ-NH₂ | 88 (0.17) | 77 |
| Ex.4 | NaHCO₃ 50mM | PEG₅ₖ-NH₂ | 99 (0.12) | 85 |
| Ex.5 | NaHCO₃ 200mM | PEG₂ₖ-NH₂ | polydisperse | - |
| Ex.6 | NaHCO₃ 50mM | PEG₁₀ₖ-NH₂ | gel | 83 |
| Ex.7 | NaHCO₃ 200mM | ELPM40 | Aggrégé à TA | - |
| Ex.8 | NaHCO₃ 50mM | PEG₅ₖ-NH₂ | 131 (0.19) | 79 |
| Ex.9 | NaHCO₃ 200mM | PEG₅ₖ-NH₂ | - | 75 |
| Ex.10 | NaHCO₃ 50mM | PEG₅ₖ-NH₂ | 92 (0.22) | 77 |
| Ex.11 | NaHCO₃ 50mM | PEG₅ₖ-NH₂ | 125 (0.33) | 72 |
| Ex.12 | NaHCO₃ 50mM | 2arms-PEG₅ₖ-NH₂ | - | - |
| Ex.13 | NaHCO₃ 50mM | 4arms-PEG₅ₖ-NH₂ | - | - |
| Ex.14 | NaHCO₃ 50mM | PSar | - | 55 |
| Ex.15 | NaHCO₃ 50mM | PGA | 99 (0.7) | - |
| Ex.16 | NaHCO₃ 50mM | Polysacc. | 414 (0.19) | - |
| Ex.17 | NaHCO₃ 50mM | PEG₅ₖ-NH₂ | - | - |
| Ex.18 | NaHCO₃ 50mM | DNA | 460 (0.26) | - |

## Revendications

1. Procédé de préparation « one pot » d'une solution aqueuse de nanoparticules de copolymères à blocs amphiphiles et comprenant des motifs polypeptidiques, ledit procédé comprenant au moins une étape (E1), dans un solvant aqueux dénué de solvant organique, consistant à mettre en présence :
- au moins un polymère hydrophile (P1) comprenant au moins une fonction amine, et
- au moins un monomère N-carboxyanhydride d'α-acide aminé hydrophobe, dans lequel la température de l'étape (E1) est comprise de -10°C à 80°C, ledit procédé étant un procédé d'auto-assemblage induit par polymérisation par ouverture de cycle du N-carboxyanhydride.

2. Procédé selon la revendication 1, dans lequel le monomère N-carboxyanhydride d'α-acide aminé hydrophobe répond à la formule (I) suivante : 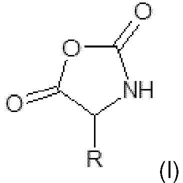 dans laquelle R représente la chaîne latérale d'un α-acide aminé, naturel ou modifié, hydrophobe, éventuellement protégé.

3. Procédé selon la revendication 1 ou 2, dans lequel le polymère (P1) est choisi dans le groupe constitué des polyéthers, des polyesters, des poly(met)arylates, des polysaccharides, des polypeptides des polypeptoides, des dérivés d'ADN et des protéines, en particulier les polymères à base d'élastine (elastin-like polypeptides (ELP)) comprenant au moins une fonction amine, et est de préférence choisi parmi les poly(oxyde d'éthylène)s comprenant au moins une fonction amine.

4. Procédé selon l'une quelconque des revendications 1 à 3, dans lequel le polymère (P1) répond à la formule suivante : 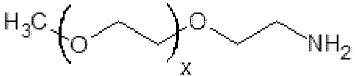 dans laquelle x est compris de 16 à 500.

5. Procédé selon l'une quelconque des revendications 1 à 4, dans lequel le solvant aqueux est de l'eau ou un tampon.

6. Procédé selon l'une quelconque des revendications 1 à 5, dans lequel le solvant aqueux comprend en outre une solution tampon comprenant un sel inorganique à des concentrations allant de 0,01 M à 1 M, notamment choisi dans le groupe constitué des solutions d'hydrogénocarbonate de sodium et des solutions tampons phosphates.

7. Procédé selon l'une quelconque des revendications 1 à 6, dans lequel le pH du solvant aqueux est compris de 2 à 12.

8. Procédé selon l'une quelconque des revendications 1 à 7, dans lequel la température de l'étape (E1) est comprise de 0°C à 4°C.

9. Procédé selon l'une quelconque des revendications 1 à 8, dans lequel l'étape (E1) est effectuée sous agitation à partir d'une dispersion du monomère N-carboxyanhydride d'α-acide aminé hydrophobe.

10. Procédé selon l'une quelconque des revendications 1 à 9, dans lequel la solution aqueuse de nanoparticules de copolymères à blocs amphiphiles obtenue à l'issue de l'étape (E1) est mise en contact ultérieurement avec un deuxième monomère N-carboxyanhydride d'α-acide aminé hydrophobe, identique ou différent de celui de l'étape (E1), ce qui permet d'obtenir une solution aqueuse de nanoparticules modifiées de copolymères à blocs amphiphiles modifiés.

## Patentansprüche

1. Verfahren zur "One-Pot"-Herstellung einer wässrigen Lösung von Nanopartikeln aus amphiphilen Blockcopolymeren und das Polypeptid-Einheiten umfasst, das Verfahren umfassend mindestens einen Schritt (E1) in einem wässrigen Lösungsmittel, das frei von organischen Lösungsmitteln ist, der darin besteht, Folgendes bereitzustellen:
- mindestens ein hydrophiles Polymer (P1), umfassend mindestens eine Aminfunktion, und
- mindestens ein hydrophobes α-Aminosäure-N-Carboxyanhydrid-Monomer, wobei die Temperatur in Schritt (E1) zwischen -10 °C und 80 °C liegt, wobei das Verfahren ein Verfahren zur durch Polymerisation induzierten Selbstorganisation durch Ringöffnung von N-Carboxyanhydrid ist.

2. Verfahren nach Anspruch 1, wobei das hydrophobe α-Aminosäure-N-Carboxyanhydrid-Monomer der folgenden Formel (I) entspricht: 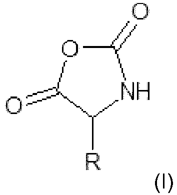 wobei R die Seitenkette einer natürlichen oder modifizierten, hydrophoben, gegebenenfalls geschützten α-Aminosäure darstellt.

3. Verfahren nach Anspruch 1 oder 2, wobei das Polymer (P1) ausgewählt ist aus der Gruppe, bestehend aus Polyethern, Polyestern, Poly(met)arylaten, Polysacchariden, Polypeptiden, DNA-Derivaten und Proteinen, insbesondere Polymeren auf Elastinbasis (elastin-like polypeptides (ELP)), umfassend mindestens eine Aminfunktion, und vorzugsweise ausgewählt ist aus Poly(ethylenoxid)en , umfassend mindestens eine Aminfunktion.

4. Verfahren nach einem der Ansprüche 1 bis 3, wobei das Polymer (P1) der folgenden Formel entspricht: 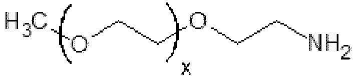 wobei x zwischen 16 und 500 liegt.

5. Verfahren nach einem der Ansprüche 1 bis 4, wobei das wässrige Lösungsmittel Wasser oder ein Puffer ist.

6. Verfahren nach einem der Ansprüche 1 bis 5, wobei das wässrige Lösungsmittel ferner eine Pufferlösung umfasst, umfassend ein anorganisches Salz in Konzentrationen von 0,01 M bis 1 M, insbesondere ausgewählt aus der Gruppe, bestehend aus Natriumhydrogencarbonatlösungen und Phosphatpufferlösungen,

7. Verfahren nach einem der Ansprüche 1 bis 6, wobei der pH des wässrigen Lösungsmittels zwischen 2 und 12 liegt.

8. Verfahren nach einem der Ansprüche 1 bis 7, wobei die Temperatur in Schritt (E1) zwischen 0 °C und 4 °C liegt.

9. Verfahren nach einem der Ansprüche 1 bis 8, wobei der Schritt (E1) unter Rühren aus einer Dispersion des hydrophoben α-Aminosäure-N-Carboxyanhydrid-Monomers durchgeführt wird.

10. Verfahren nach einem der Ansprüche 1 bis 9, wobei die aus Schritt (E1) erlangte wässrige Lösung aus Nanopartikeln aus amphiphilen Blockcopolymeren anschließend mit einem zweiten Monomer N-Carboxyanhydrid einer hydrophoben α-Aminosäure, das gleich oder verschieden von dem im Schritt (E1) ist, in Kontakt gebracht wird, wodurch eine wässrige Lösung von modifizierten Nanopartikeln aus modifizierten amphiphilen Blockcopolymeren erhalten wird.

## Claims

1. A "one pot" method for preparing an aqueous solution of nanoparticles of amphiphilic block copolymers and comprising polypeptide units, said method comprising at least one step (E1) in an aqueous solvent free of organic solvent, consisting of bringing together:
- at least one hydrophilic polymer (P1) comprising at least one amine function, and
- at least one hydrophobic α-amino acid N-carboxyanhydride monomer
wherein the temperature of the step (E1) is from -10°C to 80°C, the said method being a self-assembly method induced by ring-opening polymerisation of N-carboxyanhydride.

2. The method according to claim 1 wherein the hydrophobic α-amino acid N-carboxyanhydride monomer has the following formula (I): 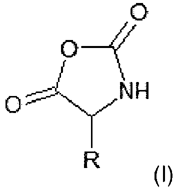 where R is the side chain of an optionally protected, natural or modified, hydrophobic α-amino acid.

3. The method according to claim 1 or 2, wherein the polymer (P1) is selected from the group consisting of: polyethers, polyesters, poly(meth)acrylates, polysaccharides, polypeptides, polypeptoids, DNA and protein derivatives, in particular elastin-like polypeptides (ELPs) comprising at least one amine function, and it is preferably selected from among poly(ethylene oxides) having at least one amine function.

4. The method according to any of claims 1 to 3, wherein the polymer (P1) has the following formula: 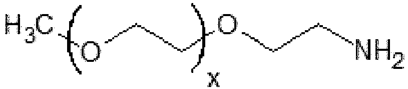 where x is from 16 to 500.

5. The method according to any of claims 1 to 4, wherein the aqueous solvent is water or a buffer.

6. The method according to any of claims 1 to 5, wherein the aqueous solvent also comprises a buffer solution comprising an inorganic salt at concentrations ranging from 0.01 M to 1 M, selected in particular from the group consisting of sodium hydrogen carbonate solutions and phosphate buffer solutions.

7. The method according to any of claims 1 to 6, wherein the pH of the aqueous solvent is from 2 to 12.

8. The method according to any of claims 1 to 7, wherein the temperature at step (E1) is from 0°C to 4°C.

9. The method according to any of claims 1 to 8, wherein step (E1) is conducted under agitation from a dispersion of the hydrophobic α-amino acid N-carboxyanhydride monomer.

10. The method according to any of claims 1 to 9, wherein the aqueous solution of nanoparticles of amphiphilic block copolymers obtained after step (E1) is subsequently contacted with a second hydrophobic α-amino acid N-carboxyanhydride monomer, the same or differing from the one at step (E1), which allows an aqueous solution to be obtained of modified nanoparticles of modified amphiphilic block copolymers
